# EUROPEAN PATENT APPLICATION

(11) **EP 1 186 319 A1**
(43) Date of publication of application: **13.03.2002**
(21) Application number: 00119741.7
(22) Date of filing: 08.09.2000
(51) Int. Cl.: A61P 35/00, A61P 37/00, A61P 17/02, C07K 14/47, C07K 16/18

(54) **Use of serum response factor (SRF) modulator for treating SRF related diseases**

(71) Applicant: Nordheim, Alfred, Prof. Dr., 72135 Dettenhausen (DE)
(72) Inventor: Nordheim, Alfred, Prof. Dr., 72135 Dettenhausen (DE)
(74) Representative: Patentanwälte Ruff, Wilhelm, Beier, Dauster & Partner

(57) **Abstract**

The invention relates to the use of an active agent influencing the expression and/or function of SRF, SRF variants and/or members of the SRF signal transduction pathway for the preparation of a therapeutic drug or a pharmaceutical composition for the treatment of disturbances or illnesses that are linked with SRF-related cellular malfunctions. Furthermore, the invention relates to the use of a substance detecting the above signal elements for the diagnosis of disturbances or illnesses linked with SRF-related cellular malfunctions, a diagnostic kit comprising such substances and a cell line which can be used for screening and identifying active agents or substances influencing the above mentioned signal elements.

## Description

The invention relates to the use of an active agent influencing the expression and/or function of SRF, SRF variants and/or members of the SRF signal transduction pathway for the preparation of a therapeutic drug or a pharmaceutical composition for the treatment of disturbances or illnesses that are linked with SRF-related cellular malfunctions. Furthermore, the invention relates to the use of a substance detecting the above signal elements for the diagnosis of disturbances or illnesses linked with SRF-related cellular malfunctions.

SRF is a widely expressed nuclear protein. It acts as a transcription factor which regulates the expression of many target genes (Johansen, F.E., and Prywes, R. 1994. Biochim Biophys Acta 1242: 1-10; Treisman, R. and Ammerer, G. 1992. Curr Opin Genet Dev 2: 22-226). SRF belongs to the family of MADS-box proteins (Schwarz-Sommer, Zl, Huijser, P., Nacken, W., Saedler, H. and Sommer, H. 1990. Science 250: 931-936), members of which are found in eukaryotic systems ranging from yeast to man (Shore, P. and Sharrocks, A.D. 1995. Eur J Biochem 229: 1-13). The human SRF protein is 508 amino acids in size, however, several splice variants of SRF have been reported recently (Kemp, P.R. and Metcalfe, J.C. 2000.

Biochem J 345: 445-451). SRF binds as a homodimer to DNA segments containing palindromic or near-palindromic CC(A/-T)₆GG sequences. SRF can act as a transcriptional acitvator of target genes, either alone or upon recruitment of partner proteins. Functionally, SRF can be subdivided into individual domains that encode specific activities, i. e. DNA binding, homo-dimerisation, interaction with partner proteins, and transcriptional activation (Johansen, F.E. and Prywes, R. 1993. Mol Cell Biol 13: 4640-4647; Johansen, F.E. and Prywes, R. 1995. Biochim Biophys Acta 1242: 1-10).

SRF is expressed very broadly. SRF mRNA and protein has been detected in all established cell lines investigated Biesida, E., Hamamori, Y., Kedes, L. and Sortorelli, V. 1999. Mol Cell Biol 19: 2577-2584), including neuronal cells and different types of muscle cell, including muscle cell lineages (striated and smooth muscle tissues) and neuronal tissues.

The activity of SRF can be regulated at several levels, like
(i) Differential cellular expression,
(ii) Differential splicing,
(iii) Protein stability
(iv) Post-translational modification of SRF as phosphorylation and glycosylation,
(v) Nuclear translocation of SRF,
(vi) Association with partner proteins, and
(vii) DNA binding.

The serum response element (SRE) was originally defined as the region of near perfect dyad symmetry, located at around-300 in the human c-fos promoter, which mediated the rapid serum- or growth factor-induced activation of the c-fos gene. This element contains the binding site for SRF.

The demonstration of SRF's ability to recruit ternary complex factors (TCFs) to a segment within the c-fos promoter immediately 5' of the SRE (Schroter, H., Mueller, C.G., Meese, K. and Nordheim, A. 1990. EMBO J 9: 1123-1130), and the realization that this protein family acts as an important mediator of serum-induced MAP kinase signalling (Gille, H., Sharrocks, A.D. and Shaw, P.E. 1992. Nature 358: 414-417), broadened the description of an SRE to representing an SRF binding site that additionally enables the recruitement of SRF partner proteins (Treisman, R. 1992. Trends Biochem Sci 17: 423-426). In fact, the intervening distance between the SRF and TCF binding sites can be quite flexible, as seen naturally in the Egr-1 promoter or as determined experimentally (Treisman, R., Marais, R. and Wynne, J. 1992. EMBO J 11: 4631-4640).
In contrast to the SRE, the CArG-boxes, as identified first in the β-actin promoters specifically expressed in muscle cells, represent SRF binding sites lacking immediately adjacent TCF binding sites. However, cooperative interaction of CArG box-bound SRF with muscle cell-specific transcription factors, such as the Nkx-2.5 protein, also requires neighbouring DNA binding sites for these SRF partner proteins. Analysis of the β-actin promoter revealed the first examples of natural CArG-boxes which did not represent CArG-box consensus sites but rather CArG-box variant sites. It appears that different sequences of CArG-box variant sites may determine the combinatorial interactions between SRF and different muscle-specific transcription factors bound nearby.

It is the object of the invention to convert prior and new scientific insights into therapeutic and diagnostic uses. This object is solved by the subject-matter of claims 1, 2, 12, 13, 15, 16, 17 and 18. Preferred embodiments are given in the dependent claims 3 to 11, 14 and 19. The wording of all these claims is hereby incorporated into the content of the description by reference.

Surprisingly it was found that SRF and SRF targeted genes are involved in the regulation of cellular activities, including cell architecture and cellular substructures, intracellular signaling, cell survival due to the activation of anti-stress genes like Bcl-2 as also cell-cell and cell-substrate interactions. Therefore, SRF-related cellular malfunctions lead to the formation of several disturbances or illnesses.

According to the invention, at least one active agent is used for influencing, particularly stimulating the expression and/or function of SRF, SRF variants and/or members of the SRF signal transduction pathway in eukaryotic cells for the treatment of disturbances or illnesses that are linked with SRF-related cellular malfunctions. This in particular also covers the use of such active agent for producing a corresponding medicament or a corresponding pharmaceutical composition. According to the present invention, the active agent can optionally be used in the form of its pharmaceutically acceptable salts and optionally together with a pharmaceutically carrier. This active agent can be e. g. a genetically modified mutant of SRF, SRF variants and/or members of the SRF signal transduction pathway. Using molecular biology techniques it is e. g. possible to produce an active agent which has an increased/decreased activity compared to the physiological aquivalent or to produce the naturally occuring signal elements, including SRF itself.

According to the invention, at least one substance can be used to detect the expression and/or function of SRF, SRF variants and/or members of the SRF signal transduction pathway in eukaryotic cells for the diagnosis of disturbances or illnesses that are linked with SRF-related cellular malfunctions. For instance it is possible to detect genetical disturbances which are linked with an increased/decreased production of SRF or gene products which are targeted by SRF, SRF variants or members of the SRF signal transduction pathway. The substance which is used to detect the expression and/or function of SRF, SRF variants and/or members of the SRF signal transduction pathway can be e. g. an antibody targeted against SRF, SRF variants and/or members of the SRF signal transduction pathway and which can be used in a method to detect said protein, e. g. ELISA (Enzyme-Linked Immuno Sorbent Assay) or another method known by those skilled in the art. Another substance which can be used are oligonucleotides, that can be used by PCR technique known by those skilled in the art. Examples will be given by the invention.

In one preferred embodiment of this invention the SRF-related cellular malfunction is a malfunction of structural components of the cellular cytoskeleton. These structural components of the cytoskeleton are focal adhesion plugs, adherence junction, stress fibers and/or cytoskeletal actin network. These structural components influence the cell architecture, cellular substructures and cellular activities. For instance, the actin cytoskeleton is involved in intracellular signalling processes. It is also known that said structural components of the cytoskeleton are involved in cellular migration therefore in misexpression altered cellular migration processes. They are needed for the cellular adhesion to extracellular substrates, to other cells or to components of the extra cellular matrix. Therefore, disturbances or illnesses in that structural components will result in diseases like tumor invasion, tumor metastasis, auto-immun diseases, disturbances of wound healing, lymphocyte homing, disturbances of immun defense mechanisms, myopathies, and cardiac cell death syndroms.

In one preferred embodiment of this invention, the active agent used is preferably targeted against at least SRF, SRF variants and/or members of the SRF signal transduction pathway. Preferred members of this SRF signal transduction pathway are Arl-4, Bcl-2, Bmp2, Bra(T), Cathepsin B, Cathepsin L, IGFBP-6, Integrin β, Keratin 16 and Keratin 18, LIM-K2, MKK6, the gene for oxidative stress induced protein, TDAG15, TGF β-R type 1, uPA-R and Zyxin. In the use according to the invention, the above mentioned genes can be targeted alone by the active agent, but there can also be a random combination of two, or three or even more different members of the SRF signal transduction pathway which are targeted by the active agent. The active agent can modulate the activity of said SRF target genes. Expression or misexpression of any target gene can contribute at least in part to the formation of diseases like metastatic cancer which is influenced by the gene uPA-R, diseases like chronic renal failure, cancer, and various hypoglycaemias influenced by the gene IGFBP-6, cancer metastasis and disturbances of wound healing events influenced by the gene Integrin β, disturbances of wound healing influenced by the expression of the gene Keratin 16 and Keratin 18, diseases which are influenced by migratory processes important for tumor invasion and metastasis, lymphocyte homing, and disturbances of the wound healing influenced by the gene Zyxin. It is important to note that the expression of the genes Cathepsin B and Cathepsin L influences inflammatory diseases and non-metastatic as also metastatic cancers.

As is mentioned above, the active agent or substance may be targeted against SRF, SRF variants and/or one member of the SRF signal transduction pathway. Furthermore, it is also possible that an activator, inhibitor, regulator and/or biological precursor of SRF, SRF variants and/or members of the SRF signal transduction pathway is targeted and/or influenced by the active agent. These activators, inhibitors, regulators, and/or biological precursors may be e. g. kinases which are known to be involved in the regulation of the activity of proteins, additional transcriptional factors responsible for the expression level of SRF, SRF variants and/or SRF members of the signal transduction pathway, or even up to date unknown compounds which can be influenced by the active agent or substance.

According to the invention it is possible to use known or also novel active agents or substances. In one preferred embodiment of the invention the active agent is a polynucleotide encoding a peptide, preferably a polypeptide, which influences, preferably stimulates the expression of SRF, SRF variants and/or members of the signal transduction pathway. This peptide can be e. g. SRF, SRF variants, the gene products mentioned above and other peptides known to those who are skilled in the art. Optionally, the active agent or substance is a peptide, preferably a polypeptide, which influences, preferably stimulates the expression of SRF or SRF variants and/or members of the SRF signal transduction pathway.

The invention comprises a pharmaceutical composition or a medicament which contains at least one active agent influencing, preferably stimulating the expression and/or function of SRF, SRF variants, and/or members of the SRF signal transduction pathway in eukaryotic cells and optionally further comprising a pharmaceutically acceptable carrier. Optionally, the active agent influences, preferably stimulates the expression and/or function of activators, inhibitors, regulators and/or biological precursors of SRF, SRF variants and/or members of the SRF signal transduction pathway in eukaryotic cells and optionally further comprising a pharmaceutically acceptable carrier. Relating to the individual features of such composition of a medicament, reference is made to the corresponding description text above.

The invention comprises further a diagnostic kit. These diagnostic kit comprises at least one substance detecting the expression and/or function of SRF, SRF variants and/or members of the SRF signal transduction pathway in eukaryotic cells. The substance can be an antibody, oligonucleotide etc. Reference is made to the description above relating the individual features of such a substance.

In a particularly preferred embodiment of the present invention, genetically engineered embryonic or somatic stem cell, modified at the SRF locus, preferably by fully or in part deletion of one, especially both SRF alleles or by replacement of one, especially both SRF alleles with mutated versions of SRF encoding SRF proteins with altered activity, or a cell derived from said cell is claimed. These cells can be modified by standard molecular biology techniques. The technique used depends on the known succesful techniques, and they are known to those skilled in the art. One example for a succesful modification of stem cells is given by the invention.

In another aspect of the invention a method is provided to screen for new SRF target genes. This method is characterized in that stem cells which were genetically engineered and modified at their SRF locus according to the description above are used.

Finally, in a most preferred aspect according to the invention it is provided a method to screen and identify an active agent or substance that can be used to influence, particularly stimulate the expression and/or function of SRF, SRF variants, members of the SRF signal transduction pathway or activators, inhibitors, regulators and/or biological precursors of SRF, SRF variants and/or members of the SRF signal transduction pathway. The method is characterized in that the influence of compounds on the expression and/or function of SRF, SRF variants and/or members of the SRF signal transduction pathway are tested, wherein optionally, stem cells according to the invention can be used.

The described features and further features of the invention can be gathered from the following description of preferred embodiments in conjunction with the subclaims. The individual features can be implemented separately or in the form of subcombinations.

### Materials and methods

### Antibodies

Primary antisera were used which recognized the following antigens:
FAD (Transduction Lab., Lexington, KA), vinculin , a-actinin (both from Sigma, St.Louis, MO), Actin (Accurate, Westbury, NY), E-cadherin (gift of H.Beug, IMP Vienna), β-catenin (gift of P. Hausen, MPI Tübingen), zyxin (gift of J. Wehland, GBF Braunschweig), VP16 (Santa Cruz).

### Plasmids

To obtain pCS2+/hSRF-VP16, a 1,8 kb Ncol/Xbal fragment (Ncol site filled-in) was isolated from pKD24 (Dalton, S. and Treisman, R. 1992. Cell 68: 597-612), and the fragment was subcloned into the vector pCS2+ (gift of R.Rupp, MPI Tübingen) linearized with Stul/Xbal.
SRF M-VP16 was generated from pCS2+/hSRF-VP16 by removing a fragment from the Bglll to the Bbsl. site. The remaining vector sequences were religated with an annealed oligonucleotide of the following sequence: 5'-GG GGC GAA GAG ACA-3' (upper strand); 5'-G ATC TGT CTC TTC G-3' (lower strand).

### ES cells

Germline competent D3 and E14 ES cells were provided by R. Jaenisch (Whitehead Institute) and K. Rajewsky (Cologne), respectively. Srf(-/+) ES cells, derived from the E14 line, were described previously (Arsenian, S., Weinhold, B., Oelgeschläger, M, Rüther, U. and Nordheim, Alfred. 1998 EMBO J 17: 6289-6299). Selection for enhanced G418 resistance and characterization of Srf(-/-) ES cells, as well as the generation of Srf(-/-)^{rescue} cells, is described later.

### ES cell culture conditions

General growth conditions. ES cells were kept without feeders on gelatin-coated dishes in DMEM medium, containing 4.5 g/l glucose and 3.7 g/l NaHCO₃, supplemented with 2mM L-glutamine, 100 U/ml penicillin, 100g/ml streptomycin, 0.1 mM mercaptoethanol, 15 % fetal bovine serum (FCS) and 1000 U leukemia inhibitory factor (LIF) (referred to as complete medium). Cultivation was at 37°C in a humidified atmosphere at 7.5 % CO₂ and cells were passaged every 2-3 days.

Selection of Srf(-/-) ES cells. ES cells were derived from the Srf(-/+) clone (clone 226) that had been used for germ line transmission (Arsenian et al., 1998). 3 x 10⁶ Srf(-/+) cells were seeded with a density of 3 x 10⁵ per 10 cm dish in complete medium. Two days after seeding, selection was applied by addition of 10 mg/ml G418. Medium was exchanged every two days. After 21 days, 100 surviving single cell-derived ES cell colonies were picked and propagated. Among them, five clones were genotyped by PCR and confirmed by Southern blotting to be Srf(-/-). Three clones, named 226-77, 226-81 and 226-100, were used for further analysis. One clone, 226-99 Srf(-/+), survived the selection protocol, yet was found to be heterozygous for the mutant Srf allele. We speculate this clone to contain an additional copy of the neo resistance gene.

### ES cell culture conditions for immediate-early gene induction.

For serum-withdrawal experiments, cells were seeded and grown for 12 h in complete medium for optimal attachment. The medium was then exchanged and cells were kept for 24-38 h in complete medium lacking FCS. The expression of immediate-early genes was assayed in serum-withdrawn and non-withdrawn cells before and 10, 30, 60 or 180 minutes after reading of 15 % FCS or 100 mg/ml phorbolester TPA (final concentrations), respectively. Same was performed for starvation experiment.

In vitro differentiation conditions. For differentiation experiments, cells were seeded in monolayers on gelatin-coated dishes and kept overnight in complete medium. The first day after seeding was defined to be day 0 of differentiation. The medium was then exchanged by complete medium lacking LIF or complete medium without LIF, but containing either 0.8 % dimethylsulfoxide (DMSO) or 5 x 10⁻⁷ M all-trans RA, respectively. Medium was exchanged daily and cells were harvested at the indicated time points for RT-PCR analysis. The vitality was determined by trypan blue staining. The vitality of detached cells was less than 5 %.

### Northern blotting.

RNA was isolated by the guanidinium isothiocyanate method (Chomczynski, P. and Sacchi, N. 1987. Anal Biochem 162: 156-159). 20 µg of RNA were electrophoresed and transferred to Gene Screen nylon membrane (NEN, Boston). Filters were successively hybridized with [³²P]dCTP-labeled human Egr-1, v-fos, and, for standardization, with mouse fox cDNA probes (Rüther, U., Komitowski, D., Schubert, F.R. and Wagner, E.F. 1989. Oncogene 4: 861-865) under standard condidtions.

### Quantitative RT-PCR.

Total RNA preparation (RNeasy kit, QUIAGEN) and first strand cDNA synthesis (Superscript II, Gibco) were done according to the manufacturers protocols. 1 µg of total RNA treated with DNAse I was used for reverse transcription. One twentieth of the RT reaction was included in a 25 µl PCR reaction. For quantitative analysis, SYBR Green PCR technology was used (Perkin Elmer). Real time detection of the PCR product was monitored by measuring the increase in fluorescence caused by the binding of SYBR Green to double-stranded DNA with ABI PRISM 7700 Sequence Detector. In order to do relative quantification, a threshold cycle (Cₜ), the cycle at which a statistically significant increase in fluorescence occurs, was derived from the resulting PCR profiles of each sample. Cₜ is a measure for the amount of template present in the starting reaction. To correct for different amounts of total cDNA in the starting reaction, Cₜ values of an endogenous control (Hprt) were subtracted from those of the corresponding sample, resulting in ΔCₜ. The relative quantitation value is expressed as 2^{ΔCt}.
Primers used:

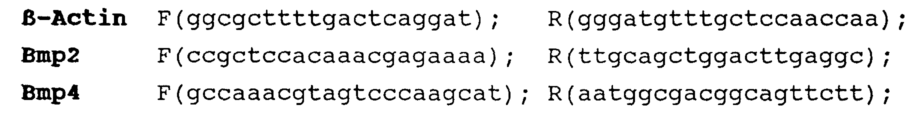

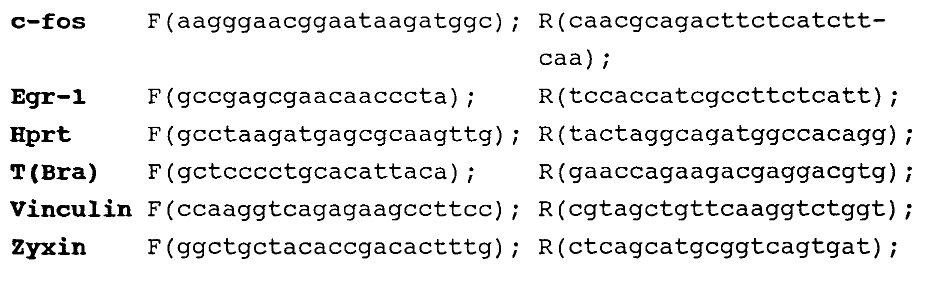

### Cell cycle analysis.

For analysis of DNA content, cells were harvested, incubated for 15 min in staining solution (0.5 mg/ml propidiumiodide, 4 mM sodium citrate, 0.03% Triton X-100), treated for 30 min with RNase A and diluted with PBS. Fluorescence intensity was determined by flow cytometry on a Becton Dickinson FACS Scan and the percentages of G1, S and G2 phase cells were calculated with the LYSIS (Becton Dickinson) or MODFIT software programs. To measure DNA incorporation, serum starved cells were cultured in the presence of 30um BrdU (Roche) for 30 minutes, trypsinized, and stored in 70% ethanol for 2 hours. Cells were rehydrated in PBS-containing 1% IFS (PBS/IFS) treated with 0.3 ml of 2M HCl at room temperature for 20 minutes, washed in PBS/IFS, neutralized with 0.1M sodium borate (pH 8.0) for 2 minutes, and washed again in PBS/IFS. BrdU was identified by labeling the cells with anti-BrdU mAb at 1:10 dilution (Roche) and goat-anti-mouse FITC. Cells were then resuspended in modified PI staining solution (0.4 mg/ml PI, 4 mM sodium citrate, 25 ug/ml RNAse A) for 15 minutes and analyzed by flow cytometry as described above.

### Scanning electron microscopy

Embryonic stem cells were grown on gelatin-coated cover slips for up to 72 h. Samples were fixed with 1.6% formaldehyde in 20 mM HEPES, 120 mM NaCl for 5 min at room temperature and for 1 hr at 4°C, post-fixed with 1% osmium tetroxide in PBS for 1 hr on ice, washed with H₂O and treated with 1% aqueous uranyl acetate for 1 hr at 4°C.
For scanning electron microscopy, ES cells grown on cover slips were dehydrated in ethanol and critical-point-dried from CO₂. The samples were sputter-coated with 8 nm gold-palladium and examined at 20 kV accelerating voltage in a Hitachi S-800 field emission scanning electron microscope. The same was done for embryoid bodies.

### Differentiation of ES cell aggregates to embryoid bodies (EBs).

For differentiation, embryonic stem cells were cultivated in hanging drops as described (Rohwedel, J., Horak, V., Hebrock, M., Fuchtbauer, E.M. and Wobus, A.M. 1995. Exp Cell Res 220: 92-100). Briefly, 600 cells in 20 µl differentiation medium (IMDM supplemented with 20 % FCS, 2 mM L-glutamine, 100 U/ml penicillin, 100g/ml streptomycin, 450 µM monothioglycerol and non-essential amino acids) were placed on the lid of bacterial petri dishes and cultivated as hanging drops for two days. EBs were then transferred into 6 cm bacterial dishes supplemented with 4 ml differentiation medium and cultivation continued for another six days.

### Genotyping of ES cells

Cells were harvested and incubated at 56°C overnight in lysis buffer (50mM Tris-HCl, pH 8.0, 100mM EDTA, 100mM NaCl, 1% SDS) containing 0.7 mg/ml proteinase K. After addition of 5M NaCl followed by centrifugation, the pellet was discarded and the DNA was precipitated from the supernatant by isopropanol treatment.
For PCR genotyping the primers **A** AGT TCA TCG ACA ACA AGC TGC GG), **B** (GAG ATT TCC ACA GAA AGC AAC GG) and **C** (TTG GGA AGA CAA TAG CAG GCA TG) were used to amplify at 94°C for 20 sec, 65°C fro 30 sec, and 72°C for 45 sec, respectively, using 35 cycles. Thereby amplifying a 360 bp fragment from the targeted allele and a 610 bp fragment from the wild type allele. For Southern blotting, DNA was restricted with Bgl II, electrophoresed, blotted to a nylon membrane and hybridized with a [³²P] dCTP labeled murine Srf fragment, detecting a 4.6 kb wild type fragment and a 5.4 kb targeted fragment.

### Electrophoretic mobility shift assays (EMSA).

Preparation of whole cell extracts and EMSA studies were performed as described previously (Heidenreich, O., Neininger, A., Schratt, G., Zinck, R., Cahill, M.A., Engel, K., Kotlyarov, A., Kraft, R., Kostka, S., Gaestel, M. and Nordheim, A. 1999. J Biol Chem 274: 14434-14443). A ³²-P labeled DNA fragment containing the c-fos SRE was used as DNA binding probe togther with up to 50% (v/v) whole cell extract prepared from ES cells. To detect specific binding of SRF to the probe, anti-SRF antiserum (rabbit lgG, Santa Cruz) was inluded for super-shift analyses.

### Western blotting.

Preparation of cell extracts was as described for EMSA, 10-20 µg of protein were resolved by SDS-PAGE on 10% polyacrylamid gels, followed by transfer to PVDF membrane. The membranes were bloced with 5% non-dry fat milk powder and subsequently probed with anti-actin (1:500 in TST) for 1 hour and HRP-conjugated goat-anti mouse IgG (1:5000 in TST, Amersham) for 30 min. After extensive washing in TST buffer, enzymatic reaction was performed with ECLplus Detection kit (Amersham) according to the manufacturers recommendations, and the blot exposed to X-ray film (Kodak). Quantification was done with Image Gauge V3.0 software.

### Semi-quantitative RT-PCR.

1µg of total RNA was used for reverse transcription using Expand™ Reverse Transcriptase kit (Boehringer Mannheim) accoring to the suppliers instructions. One-twentieth of this reaction was used for PCR amplification with specific primers. Primers and PCR annealing conditions:

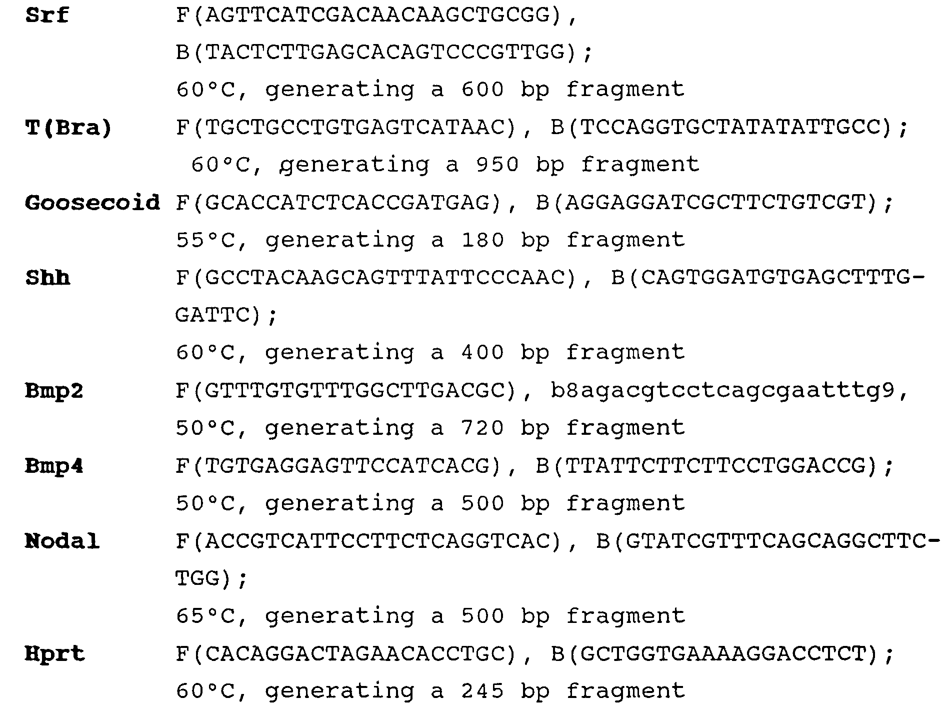

### Indirect Immunofluorescence

Day 8 embryoid bodies were fixed in 4% formaldehyde and permeabilized in 0.2% Triton-X-100 in PBS. Non-specific binding was blocked by incubating the EBs for one hour in 1% BSA in PBS at 37°C before staining with rabbit anti-laminin (1:30 in PBS; Sigma) for 1 hour at 37°C. Secondary antibody was FITC-anti-rabbit. EBs were washed four times with PBS, once in water and mounted in Moviol. Image acquisition was done with a laser scanning microscope (LSM 510, Zeiss). Alternatively, the cells were grown on gelatine-coated coverslips in complete growth medium for 48 hours, or as EBs in droplets for up to 8 days. The samples were then fixed in 4% formaldehyde and permeabilized in 0,2% Triton-X 100. Non-specific binding was blocked by incubating the cells for one hour in 1% BSA in PBS at 37°C before staining with primary antibodies for 1 hour at 37°C. Incubation with fluorescence conjugated secondary antibodies (Molecular Probes, 1:200 in 0,2% BSA) was performed for 30 min at 37°C. To visualize filamentous actin, 1 unit Oregon-green phalloidin was included into the mixture. Coverslips were washed four times with PBS, once in water, air-dried and mounted in Moviol. Image acquisition was done with LSM 510 (Zeiss).

### Sectioning of EBs and staining with toluidin.

EBs were embedded in 2% agarose, dehydrated through a graded series of ethanol and embedded in Epon. Toluidine blue stained Epon sections of 0.5 or 3 µm thickness were prepared for light microscopy.

### Atlas array hybridization

Atlas array analysis was performed as recommended by the manufacturer (Clontech). Briefly, 3µg of total RNA of undifferentiated ES cell lines were used for cDNA synthesis in the presence of ³²P-dATP. Membranes were incubated over night with the radioactively labeled cDNA and washed four times with 2x SSC, 1% SDA, twice with 0,1x SSC, 0,5% SDS. The sealed membranes were exposed to IP plates for up to five days and signals were detected with a phosphoimager.

### FACS analysis

ES cells were collected, fixed in 4% formaldehyde for 10 min, permeabilized with 0,1% Triton-X for 5 min and blocked in 1% BSA for 1 hour. After washing three times in PBS, the cells were incubated for 30 min in a mixture of 1 unit Oregon-Green phalloidin and 0,3 µM Texas-Red DNAse I (both from Molecular Probes) in 1% BSA. For a reference, incubation was performed in the absence of fluorescent dye. After washing, cells were subjected to FACScan analysis (Becton-Dickinson). Dead cells were gated out and 10⁴ cells were counted for each sample. Data analysis was done with CellQuest software (Becton Dickinson).

### Electron microscopy

Embryonic stem cells were grown on gelatine-coated cover slips for up to 72 hours or as EBs in droplets up to 8 days. Samples were fixed with 1.6% glutaraldehyde in 20 mM HEPES, 120 mM NaCl for 5 min at room temperature and for 1 hr at 4°C, postfixed with 1% osmium tetroxide in PBS for 1 hr on ice washed with H₂O and treated with 1% aqueous uranyl acetate for 1 hr at 4°C.
For transmission electron microscopy EBs were embedded in 2% agarose, dehydrated through a graded series of ethanol and embedded in Epon. Ultrathin sections were stained with uranyl acetate and lead citrate and viewed in a Philips CM10 electron microscope.

### Figure legends.

Fig 1: Proliferation kinetics, growth rates, and colony forming capabillties of Srf(-/-) ES cells.

Fig. 2: Altered cellular morphology of Srf(-/-) ES cells and their colonies.

Fig. 3: Progression through the cell cycle of Srf(-/-) ES cells.

Fig. 4: Serum withdrawal of wild type ES cells.

Fig. 5: Severe impairment of immediate early gene induction in Srf(-/-) ES cells.

Fig. 6: Genotypes and SRF protein activities of ES cells mutated at the Srf locus.

Fig 7: During in vitro differentiation of monolayer ES cells, the expression of mesodermal differentiation marker genes is impaired in cells lacking SRF.

Fig. 8: Srf(-/-) Monolayer ES cells activate mesodermal marker genes when ectopically expressing SRF or when treated with retinoic acid.

Fig. 9: Differentiaticon of Srf(-/-) ES cell aggregates leading to the formation of embryoid bodies (EBs).

Fig. 10: Cellular arrangements and cavitation in the interiors of EBs derived from ES cells of different Srf genotype.

Fig. 11: Expression of differentiation marker genes in embryoid bodies generated from ES cells of different Srf genotype.

Fig. 12: Srf(-/-) ES cells are able to give rise to teratornas containing mesodermal cell types.

Fig. 13: Srf(-/-) ES cells do not spread.

Fig. 14: Srf(-/-) ES cells are unable to form focal adhesions and actin stress fibers.

Fig. 15: Reduced actin levels in Srf(-/-) ES cells.

Fig. 16: Zyxin is down regulated in Srf(-/-) ES cells

Fig. 17: SRF-VP16 activates cytoskeletal genes

Fig 18: Impaired cell-cell interaction in embryoid bodies lacking SRF

Fig. 19: Ultrastructural analysis of cellular contacts within embryoid bodies (EBS).

### RESULTS

To investigate the contributions of SRF to cellular growth control, we generated Srf(-/-)ES cells derivad from the Srf(-/+) ES cells that were originally used for blastocyst injections and generation of Srf(-/+) mice (Arsenian, S., Weinhold, B., Oelschlager, M., Rüther, U. and Nordheim, A. 1998. EMBO J 17: 6289-6299). Such Srf(-/-) ES cells were obtained from heterozygous Srf(-/+) ES cells upon selection for elevated cellular resistance levels to the drug G418 as is described in Materials and methods. In these Srf(-/-) ES cells no protein is observed that might be antigenically related to SRF and no SRF-derived DNA binding activity can be seen.

### EXPERIMENT 1:

### Normal proliferation but impaired colony formation of Srf(-/-) ES cells.

(A) Proliferation kinetics. Growth curves of ES cell lines with the indicated Srf genotypes were generated by seeding 2000 cells at day 0 and counting total cell numbers at the indicated time points. One representative experiment of three independent ones is shown.
(B) Rates of proliferation. Semi-logarithmic plot of cell numbers as counted during the growth periods of exponential growth seen in (A). The slopes of the graphs indicate proliferation rates.
(C) Efficiencies of colony formation. 2x10³ cells each of the indicated ES cell lines were plated on gelatin-coated dishes and the resulting colonies were counted after six days of cultivation. Each value represents the mean of duplicate measurements, except for ES cell line 226-100 where four measurements are averaged.

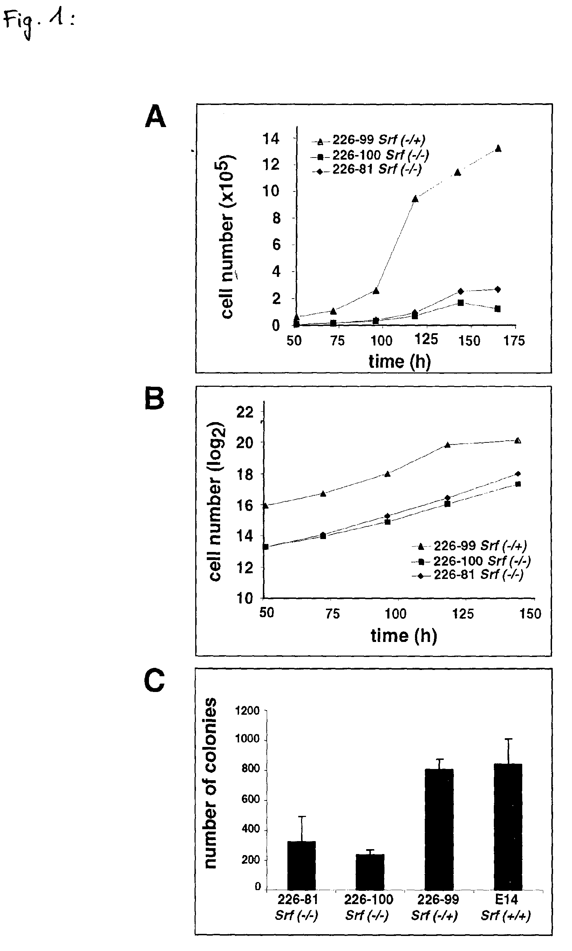

Since SRF activity appears to be essential for the growth of in vitro cultured somatic cells (Gauthier-Rouviere, C., Cavadore, J.C., Blanchard, J.M., Lamb, N.J. and Fernandez, A. 1991. Cell Regul 2: 575-588; Soulez, M., Rouviere, C.G., Chafey, P., Hentzen, D., Vandromme, M., Lautredou, N., Lamb, N., Kahn, A., and Tuil., D. 1996. Mol Cell Biol 16: 6065-6074), we sought to determine if SRF deficiency altered the growth characteristics of ES cells. We first examined the growth rates of one Srf(-/+) and two Srf(-/-) ES clones. Cells were seeded at equal densities in LIF-containing medium and their growth rates were followed by counting of total cell numbers for 7 days. Proliferation started after a lag period of one to two days in all clones. The growth curves revealed that the Srf(-/+) clone increased more rapidly in cell number than did each of the two Srf(-/-) clones (Fig 1A). However, the proliferation rates of heterozygous and homozygous mutant cell lines appeared to be similar, as judged by the slopes of the semi-logarithmic plots of cell numbers over time (Fig. 1 B). Also, no significant differences in proliferation rates were observed between wild type and Srf(-/+) ES cells. We infer from these data that SRF does not contribute in an important way to the rates which ES cells proliferate.

Since the Srf(-/+) and the two Srf(-/-) clones grew at comparable rates yet differed in increase in total cell number over time, we speculated that differences in plating efficiency with each passage contributed significantly to the differences in growth. To investigate the efficiencies of colony formation subsequent to plating, we seeded identical numbers of ES cells of different Srf genotype and counted the number of colonies formed after six days of culturing. Both Srf(-/-) ES clones displayed significantly reduced efficiencies in colony formation as compared to the wild type and Srf(-/+) clones (Fig. 1C). We believe that this difference is the prime cause for the differences in cumulative cell number over time in cultures of ES cells containing or lacking SRF.

### EXPERIMENT 2:

### Altered morphologies of colonies derived from Srf(-/-) ES cells.

Srf(-/+) (left panels) or Stf(-/-) (right panels) ES cells were cultivated for 48h in complete growth medium, trypsinized and subsequently plated for either three hours (A) or 72 hours (B,C). The obtained cell and colony morphologies were inspected by light microscopy (A,B) or scanning electron microscopy (C). Cell protrusions (marked by arrows in (A) are observed very frequently in the homozygous mutant ES cells but were found rarely in Srf(-/+) cells.

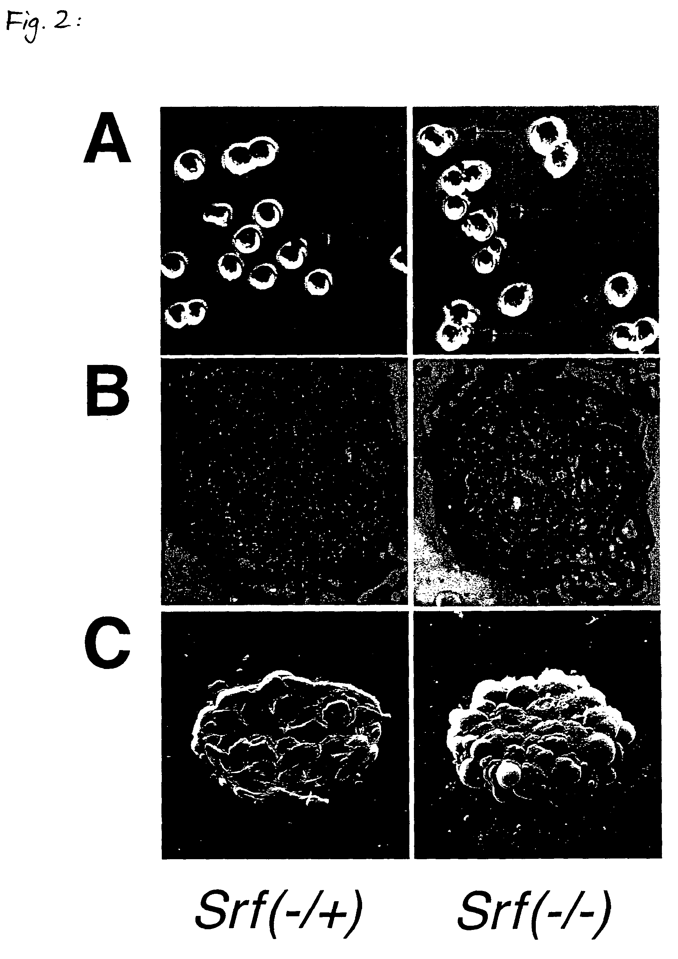

The observed differences in plating efficiency of Srf(-/-) ES cell clones (Fig. 1C) warranted a closer inspection of their morphologies. Light microscopic analysis of ES cells revealed protrusions on many of the SRF-deficient cells (Fig. 2A, right panel), features rarely seen with Srf(-/+) ES cells (Fig. 2A, left panel) or with wild type ES cells. After 72 hours of culture, colonies of cells deficient in SRF were less compact and appeared to display less cell-cell interaction (Fig. 2B). This was confirmed by scanning electron microscopy (SEM), which demonstrated that individual cells within an Srf(-/-) ES colony established fewer contacts with neighbouring cells than those within an Srf(-/+) ES colony (Fig. 2C). We speculate that the decreased plating efficiency is a direct consequence of these altered structural characteristics.

### EXPERIMENT 3:

### Cell cycle progression of Srf(-/-) ES cells.

Distribution within the cell cycle of different ES cell lines. ES cells of the indicated genotypes were harvested after cultivation under continuous, normal growth conditions for three days (A), after serum starvation for 38h (B), or after serum starvation for 38h and subsequent re-addition of serum for 24h (C). Cells were stained with propidium-iodide for determinations of DNA content and cell cycle distribution. The percentages of cells found in the G1, S and G2 phases are shown.

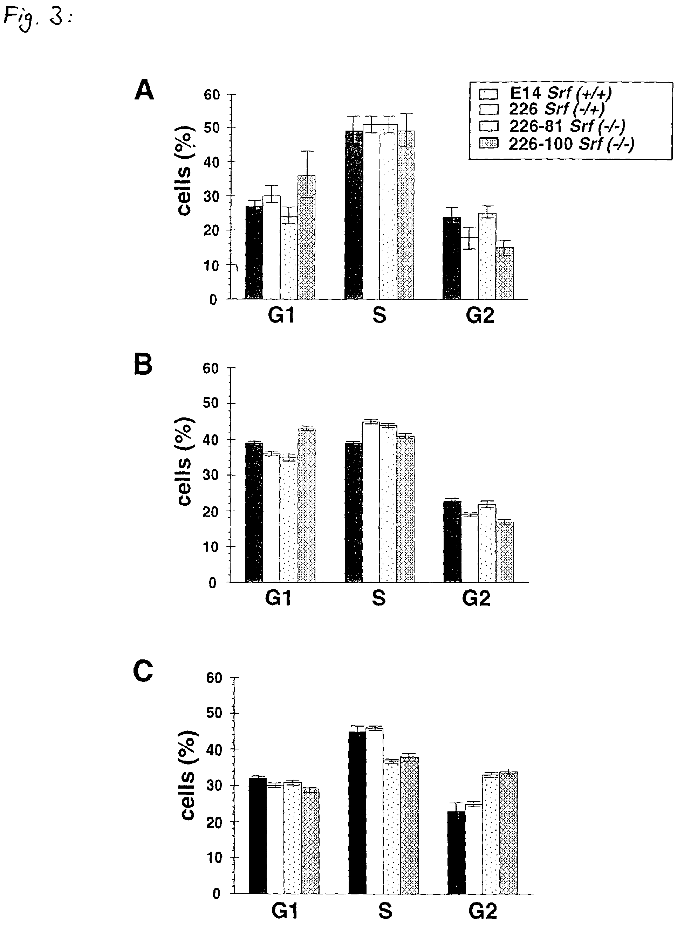

The apparently normal proliferation rate of SRF-deficient ES cells contrasted with earlier demonstrations of SRF being required for the proliferation of somatic cells. SRF is known to regulate gene activation at specific stages of the cellular growth cycle (i.a. the GO-G1 transition as quiescent cells enter into the active growth cycle, progression of cycling cells through G1, and the G2-M transition). This cell cycle-specific nature of SRF function suggested that ES cells lacking SRF may display specific alterations in the progression through the cell cycle, despite having a growth rate similar to wild type cells. The parental ES cell line E14 (Srf(+/+)), the heterozygous line 226 (Srf(-/+)), and the two homozygous lines 226-81 and 226-100 (Srf(-/-) were studied. In non-starved, normally growing cultures (Fig. 3A), the majority of cells was found in the S phase, and lower but comparable amounts of cells were found in each of the G1 and G2 phases. No obvious differences in these distributions were seen between wild type, heterozygote, or mutant ES cells. Despite the normal proliferation rate and cell cycle distribution of Srf(-/-) ES cell cultured in the presence of serum, we speculated that serum restimulation of quieseent Srf(-/-) ES cells may be altered in some manner. Thus, we analyzed the cell cycle distribution profiles of the various ES cell lines after serum withdrawal and upon subsequent stimulation with serum. In none of the ES clones did serum withdrawal lead to efficient growth arrest. After a 38h period of starvation, the percentage of G1 cells was slightly but significatly increased at the expense of S phase cells, but there was no difference between the cells of different Srf genotype (Fig. 3B). When wild type and heterozygote ES cells were re-exposed to serum for 24h (Fig. 3C), the cell cycle distribution again resembled that of asynchronously growing cells. Re-stimulation of starved mutant cells with serum, however, led to the accumulation of 10 % more of the cells in the G2/M phase of the cell cycle, a small but significant alteration in the cell cycle distribution of the cells (Fig. 3C). Thus, while SRF function does not appear to be essential for the cell cycle progression of ES cells, it may nonetheless contribute to G2/M checkpoint function, as has been previously suggested (Liu, S.H., Lee, H.H., Chen, J.J., Chuang, C.F. and Ng, S.Y. 1994. Cell Growth Differ 5: 447-455).

### EXPERIMENT 4:

### Cell cycle progression of wild type ES cells.

Wild type ES cells were labeled with BrdU for 30 minutes and harvested prior to or following 8, 16, or 24 hours of serum starvation as indicated. Cell cycle distribution (left column) was determined by staining cells for DNA content with propidiumiodide and rate of DNA synthesis (right column) was determined by immunostaining cells for BrdU incorporation (y axis) and'plotted against DNA content (x axis). The percentages of cells in the G1, S and G2 phases are indicated.

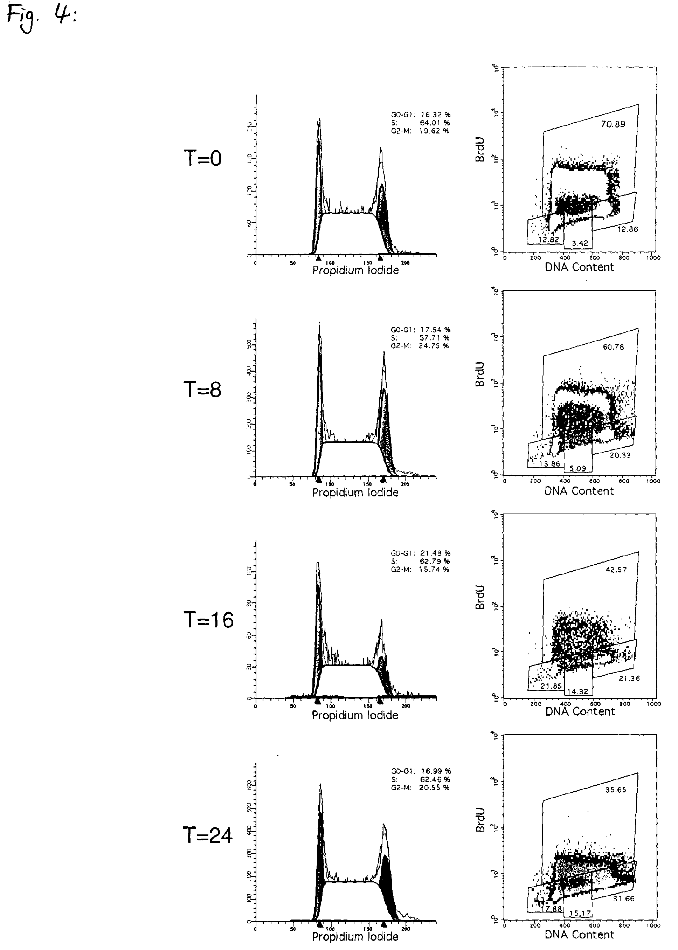

We were surprised that ES cells failed to accumulate in G1 in the absence of serum. We wished to test whether this was a characteristic of ES cells in general, and whether the ES cells are altered in any way in the absence of serum. To do so, we examined a second independently derived ES cell line, termed D3, which is also capable of germ line chimerization. As noted for E14 ES cells above, the cell cycle profile of D3 cells was not substantially altered up to 24 hours after withdrawal of serum (Fig. 4). In this experiment we also examine DNA synthesis by the uptake of the thymidine analog bromodeoxyuridine (BrdU). Although serum-starved cells retained normal cell cycle distribution, their rat of DNA synthesis gradually slowed, as evidenced by a progressive decrease in the rate of uptake of BrdU. Thus, ES cells appear to be sensitive to serum withdrawal as evidenced by a slowing in ihe rate of DNA synthesis, yet nonotheless lack the ability to undergo GO exit.

### EXPERIMENT 5:

### SRF-mediated induction of the immediate-early genes Egr-1 and c-fos insdispensable in the ES cell.

(A, B) Northern blot analysis of Egr-1 (A) and c-fos (B) RNA expression in the Srf(-/+)ES line 44 (top panel 1) or the Srf(-/-) ES line 226-100 (bottom panel) under normal growth conditions (lane 1), after serum-withdrawal for 24 hours (lane 2), or after serum-withdrawal and subsequent induction with either 15% serum (lanes 3 to 6) or 100ng/ml TPA (lanes 7 to 10) for the indicated times. Lane 11 represents a positive hybridization control. The blot probed in (A) was reprobed for c-fos. The asterisk indicates residual Egrl signal still present from prior probing of the blot. Equivalent RNA loading in eachlane was demonstrated by hybridization of the blot with a Fox probe (Rüther, U., Komitowski, D., Schubert F.R. and Wagner, E.F. 1989. Oncogene 4: 861-865).

(C-E) Quantitative RT-PCR of Egr-1 (C), c-fos (D), and vinculin (E) in ES cell lines of the indicated SRF genotype, as well as in Srf(-/-) ES cells that ectopically express human Srf cDNA (Srf(-/-)^{rescue}. Cells were either serum starved for 36 hours or serum-starved and subsequently re-stimulated with 15 % serum for the indicated times. mRNA levels were normalized to mRNA levels of the Hprt gene, and values are given as fold induction over non-stimulated cells. Each measurement was performed in duplicate.

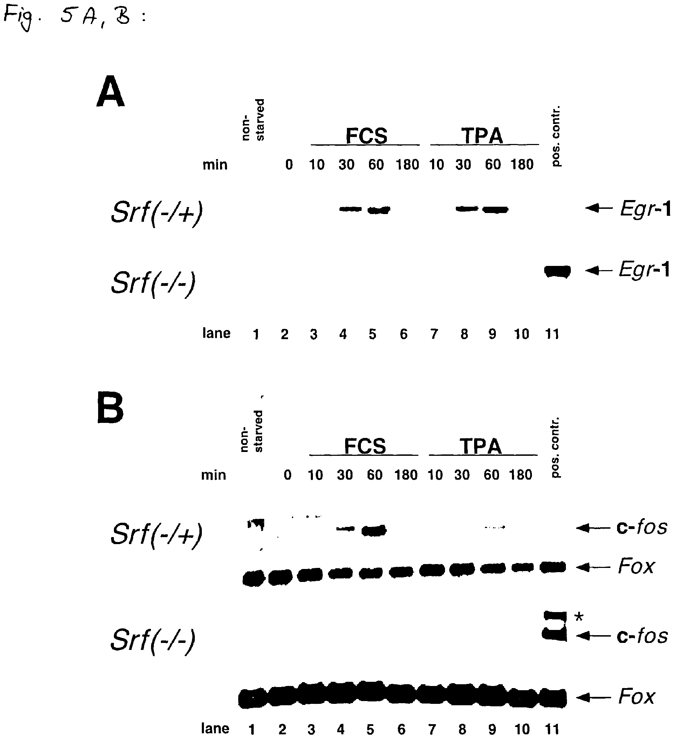

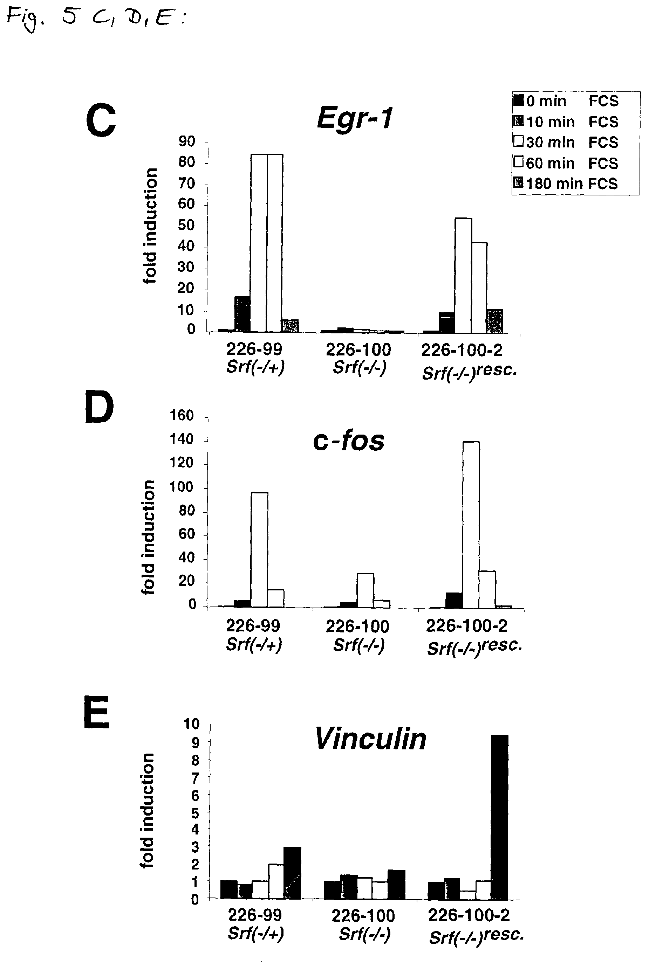

Given the relatively inefficient exit of ES cells from the active cell cycle into GO and the lack of requirement for SRF on ES cell proliferation, we wished to know whether wild type ES cells were able to mount an efficient IEG activation response. The activation of SRF complexes by the MAP kinase cascade has been implicated in the transcriptional upregulation of immediate-early genes such as c-fos and Egr-1 in the GO-G1 transition. This step is believed to be essential for resting cells to re-enter the cell cycle (Herschman, H.R. 1991. Annu Rev Biochem 60: 68-70). We therefore investigated the pattern of Egr-1 and c-fos induction following stimulation of ES cells with intact SRF function, either wild type or Srf(-/+) ES cells (Fig. 5). Serum-withdrawn ES cells were stimulated for up to 3h with either serum or the phorbol ester TPA and the resulting mRNA levels were measured over time by Northern blot analysis. ES cells containing at least one intact Srf allele were able to mount a robust IEG response, as judged by c-fos and Egr-1 gene activation profiles. A strongly contrasting result was seen with the Srf(-/-) cells. They exhibited a drastically impaired induction both Egr-1 and c-fos following serum stimulation. Whereas no induction could be seen for Egr-1 at all in the Srf(-/-) ES cells, a residual response was observed with c-fos. These data were confirmed by quantitative RT-PCR analysis. While not examined, we suggest that the induction of other IEGs is similarly compromised in these cells.

To confirm that the lack of IEG induction in the mutant ES cells was due to a deficiency o SRF, wo reintroduced the SRF protein back into these cells by ectopic expression of the cDNA from the CMV promoter. In these Srf(-/-) ES cells the levels of SRF expression are 3-5 fold higher than the endogenous protein as will be shown later. Indeed, the Srf(-/-)^{rescue} ES clone re-acquired a very efficient serumdependent induction of both c-fos and Egr-1 (Fig. 5C and D). This induction was both rapid and transient, in a manner similar to that seen in wild type ES cells expressing endogenous SRF. These data provide for the first time direct genetic evidence that SRF is an important regulator of a component of the cellular immediate-early gene activation response.

We also investigated the expression profiles of the β-Actin and Vinculin genes, both of which are known to be SRF-regulated, and do not fall into the class of transiently induced IEGs (Moiseyeva, E.P., Weller, P.A., Zhidkova, N.I., Corben, E.B., Patel, B., Jasinska, I., Koteliansky, V.E. and Critchley, D.R. 1993. J Biol Chem 268: 4318-4325). Indeed, in SRF-containing heterozygous ES cells both β-Actin and Vinculin (Fig. 5E) are stimulated 1.5 to 3 fold by serum addition. This activation, however, did not follow the rapid and transient IEG activation kinetics but rather mRNAs accumulated gradually over a three hour time period. The basal levels of β-Actin as well as the serum-stimulated induction of both β-Actin and Vinculin were reduced in Srf(-/-) ES cells. Again, reintroduction of SRF protein into the Srf(-/-)^{rescue} ES cells restored in serum-stimulated expression of both genes. In addition, the constitutive over-expression of SRF in Srf(-/-)^{rescue} ES cells was associated with elevated basal expression levels of both genes (Fig. 5E). From these data we conclude that in ES cells SRF controls the expression levels of SRE-containing genes. In doing so, it regulates rapidly responding, transiently activated IEGs, such as Egr-1 and c-fos, as well as β-Actin and Vinculin, other SRE-containing genes that do not display the same transient activation profiles.

### EXPERIMENT 6:

### Embryonic stem cells with mutated Srf loci.

(A) Genotyping by Southern blotting of ES cell lines used in this study. Genomic DNAs from the ES cell lines E14.1 Srf(+/+) (lane 1), 226 Srf(+/-) (lane 7) and 226-100 Srf(-/-) (lane 2), 226-100 Srf(-/-) plus human Srf cDNA expression construct pSGSRF1 (rescue) (lanes 3 to 5), or 226-100 Srf (-/-) plus an empty vector (vector) (lane 6), were incubated with BglII, electrophoresed and blotted. Filters were successively hybridized with either a murine Srf probe discriminating between wild type and targeted alleles (top panel), or a human Srf probe detecting the integrated human Srf cDNA (bottom panel). For hybridization control, the plasmid pSGSRF1 was used (Srf cDNA)(lane 8). Positions of the BglII fragments representing the wild type allele (4.6 kb) or the targeted allele (5.4 kb) are indicated. Lanes 3, 4, and 5 represent the "rescue" lines 226-100-2, 226-100-37, 226-100-42, respectively.
(B) Western analysis of SRF protein in extracts of ES cell lines used in this study. Reproduction of the blot (top) and its quantitation (bottom) is provided. No specific SRF Western signal is observed in the homozygous Srf(-/-) ES cell lines.
(C) SRF-mediated SRE binding activities in extracts of ES cell lines used in this study. A radiolabeled c-fos SRE oligonucleotide probe was incubated with recombinant Histagged SRF (lanes 1, 2), or protein extracts from ES cells of the different genotypes as indicated. Even lanes represent extracts with added polyclonal anti-SRF serum. SRF-containing DNA-protein complexes were found supershifted by this antiserum. No SRF-containing complexes could be detected in extracts from Srf(-/-) ES cells (lanes 7-10). Lanes 11 and 12 contained 4 µg protein extract only whereas the other cell extracts were used at 20 µg of protein.

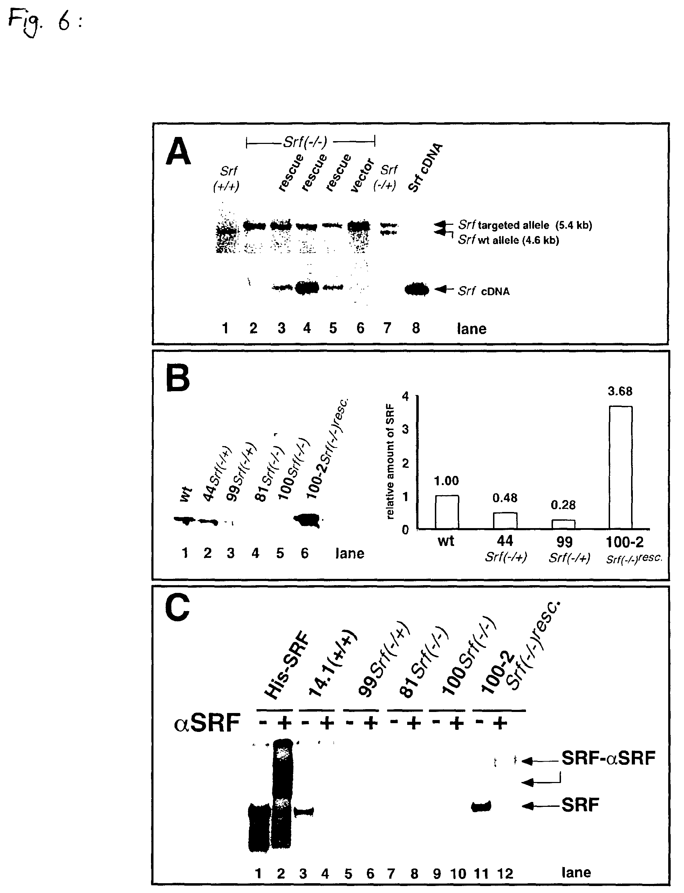

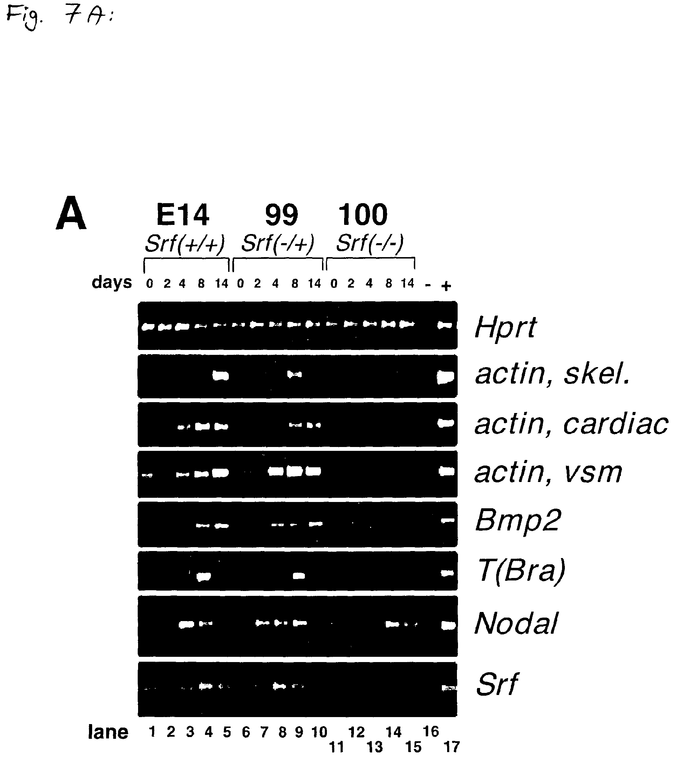

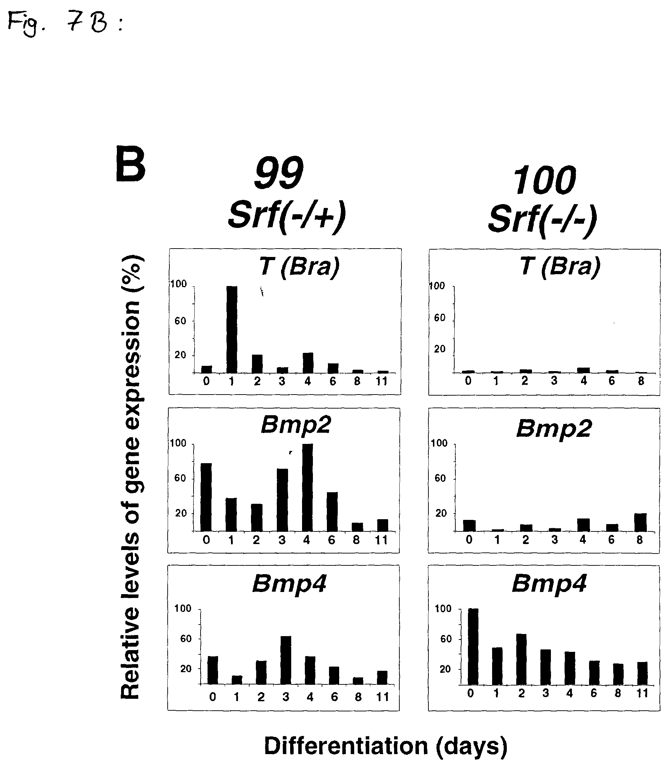

In order to investigate at the cellular and molecular level the mesodermal differentiation defect of Srf(-/-) embryos (Arsenian et al., 1998, see above) we made again use of the ES cell in vitro differentiation system. We selected homozygous Srf(-/-) ES cells from the existing hetorozygous Srf(-/+) ES cell clone (clone 226) by applying elevated Geneticin selection
(10 mg/ml). Several Srf(-/-) ES clones were selected and genotyped by PCR and Southern analysis (e.g. clones 226-77, 226-81, arid 226-1 00). Fig. 6A (lane 2) shows the genotyping by Southern blotting of one of the Srf(-/-) ES clones, 226-100. We confirmed the lack of SRF expression in Srf(-/-) ES clones by RT-PCR (see e.g. Figure 7A) and by Western blot analysis (Fig. 6B). Reduced levels of SRF protein were observed in the two heterozygous ES lines 44 and 99. Electrophoretic Mobility Shift Analysis (EMSA), also failed to detect specific SRF DNA binding activity in the Srf(-/-) cells (Fig. 6C). The Srf(-/-) ES cell clones show normal rates of proliferation but display altered cell morphologies and cell surface activities and, in addition, exhibit an impaired immediate-early gene activation response as was shown below.

SRF expresssion was rescued in these Srf(-/-) ES cell clones by the reintroduction of a CMV-driven expression vector encoding Srf cDNA. Southern blotting of three such Srf(-/-) ES cell clones (i.e. clones 226-100-2, 226-100-37, and 226-100-42) is shown in Fig. 6A. These clones have regained SRE-specific SRF DNA binding activity (Fig. 6C) as well as the ability to activate SRE-regulated target genes. We note that all our Srf(-/-)^{rescue} ES cell clones tested expressed higher than the levels of SRF protein (Fig. 6B).

### EXPERIMENT 7:

### In vitro differentiation potential of Srf(-/-) ES cells is severely impaired.

(A) Expression of differentiation markers in monolayer ES cells of different Srf genotype upon induction of in vitro differentiation by LIF withdrawal (semiquantitative RT PCR analysis). ES lines used: E14.1 (Srf(+/+) (lanes 1 to 5), 226 (Srf(-/+) (lanes 6 to 10), and 226-100 (Srf(-/-) (lanes 11 to 15). RNA expression levels of the genes indicated on the right of the figure were determined by semi-quantitative RT-PCR studies, covering a differentiation period of 14 days, as indicated. Lane 16 represents a negative RT-PCR control lane lacking cDNA in the reaction. Lane 17 represents a positive control for RTR-PCR amplification of each gene.
(B) Relative expression levels of differentiation markers in monolayer ES cells of different Srf genotype upon induction of in vitro differentiation by LIF withdrawal plus simultaneous addition of DMSO (quantitative RT-PCR analysis). ES lines used: 226-99(Srf(-/+) (left panel) and 226-100 (Srf (-/-) (right panel). Expression was followed for the time periods of differentiation (days) as indicated.

We exploited the ability of ES cells, cultivated as monolayers, to initiate an in vitro differentiation program upon withdrawal of LIF (Guan, K., Rohwedel, J. and Wobus, A. 1999. Cytotechnology 30: 211-226; Keller, G.M. 1995. Curr Opin Cell Biol 7: 862-869; Shen, M.M. and Leder, P. 1992. Cell 56: 563-572). Using Srf(+/+), Srf(-/+), and Srf(-/-) ES clones, we first followed their differentiation by morphological criteria. Endoderm-like cells formed rapidly in wild type or Srf heterozygous ES cells cultivated in the absence of LIF. Depending on the differentiation protocol, fibroblast-like cell types could be observed after 3-6 days. Subsequently, several cell types of mesodermal origin were discernable,e.g. beating cardiomyocytes and blood
islands. In contrast, Srf(-/-) ES cells grown in monolayer culture only formed an endoderm-like cell type, as judged by morphological criteria. Fibroblast-like cells or any cells of mesodermal origin, like muscle cells, were never observed, irrespective of the differentiation protocol used. We also followed RNA expression levels for various differentiation marker genes using either semi-quantitative RT-PCR (Fig. 7A; investigating 14 days of differentiation in two-day intervals) or quantitative RT-PCR (Fig. 7B; investigating 6 days of differentiation in one-day intervals). Differentiation was induced by either LIF withdrawal alone (Fig. 7A) or by LIF withdrawal plus addition of DMSO (Fig. 7B). Expression levels of Hprt and Srf served as internal controls. Bmp2, Bmp4, Nodal, T(Bra), as well as the muscle Actin genes were expressed at different times of differentiation in SRF-containing cells (Fig. 7A, lanes 1-10; 7B). Srf RNA levels stayed fairly constant during the course of differentiation. As expected for these cells, LIF withdrawal plus addition of DMSO led to an earlier induction of the mesodermal markers T(Bra) and Bmp2 than did LIF withdrawal alone (compare Fig. 7B, first and second panels from top with Fig. 7A, lanes 1 to 10). None of these genes, however, were seen activated in Srf(-/-) ES cells, except for Nodal (Fig. 7A. lanes 11-15) and Bmp4 (Fig. 7B, third panel from top).

### EXPERIMENT 8:

### Srf(-/-)^{rescue} cells, ectopically over-expressing SRF, induce mesodermal marker gene expression.

(A) Gene expression patterns in ES cells of different Srf genotype, including Srf "rescued" cells. ES lines used were: 226-100-2 (Srf(-/-)^{rescue} (lane 1), 226-100-37 (Srf(-/-)^{rescue} (lane 2), 226-100-42 Srf(-/-)^{rescue} (lane 3), 226-100 (Srf(-/-) (lane 4), and 44 (Srf(-/+) (lanes 5 and 6). LIF removal was only done with the cells investigated in lane 6. Analysis was performed as described in Fig. 7A. Lane 7 represents a negative RT-PCR control lacking cDNA in the reaction. Of the three Srf(-/-)^{rescue} ES lines, 226-100-2 expressed highest levels of SRF (i.e. approx. 4-fold endogenous levels).
(B) Expression of differentiation markers in monolayer ES cells of different Srf genotype upon induction of in vitro differentiation by LIF withdrawal plus simultaneous addition of retinoic acid. ES lines used were: 44 Srf(-/+), lanes 1 to 7) and 226-81 (Srf(-/-)(lanes 8 to 14). Analysis was performed as described in Fig. 7A.

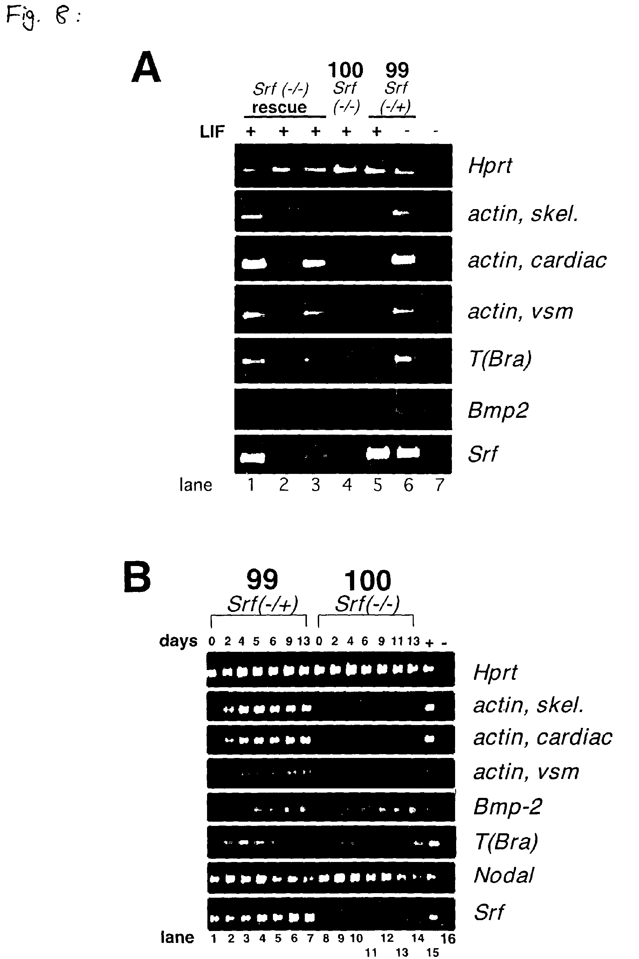

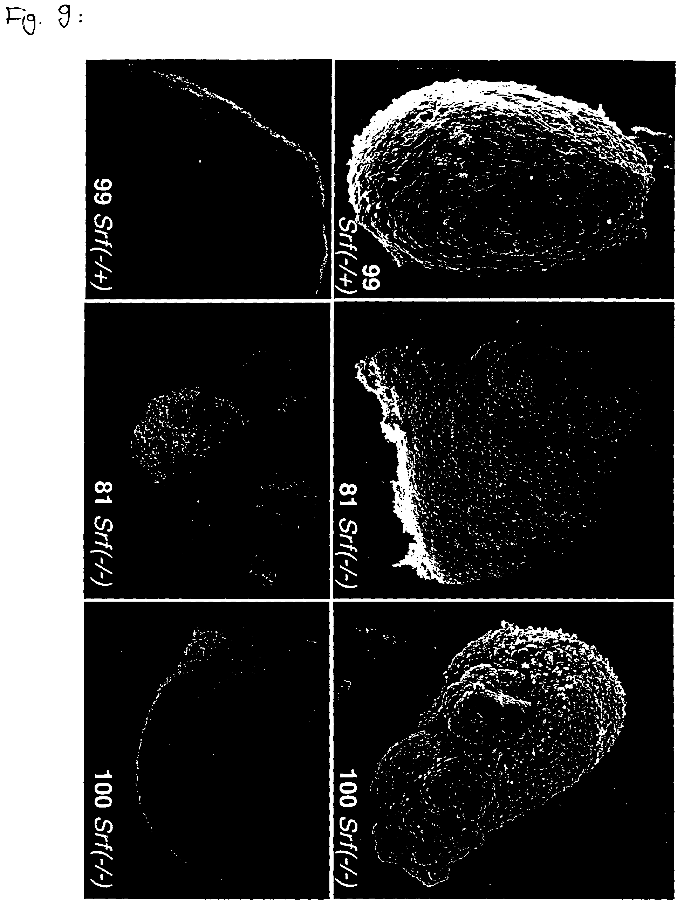

Further substantiation of the regulatory influence of SRF on the expression of differentiation marker genes can be derived from the Srf(-/-) ES clones which ectopically express SRF protein (Fig. 6B). These rescued cells exhibited spontaneous differentiation behaviour when grown as monolayers in vitro and, at variable levels, displayed elevated RNA expression of the differentiation markers investigated (Fig. 8A). This indicates that Srf(-/-) ES cells have not lost irreversibly their ability to spontaneously activate mesodermal marker genes and that overexpression of SRF alone can poise ES cells to activate mesodermal gene expression programs.

RA induces mesodermal marker genes in Srf(-/-) cells grown as monolayers. Similar to the effects of DMSO treatment on gene expression in heterozygous Srf(-/+)ES cells grown in monolayer, RA treatment combined with LIF withdrawal induced the mesodermal lineage differentiation marker genes more rapidly than did LIF withdrawal alone (compare Fig. 7A, lanes 6-10, with Fig. 7B, left panel, and with Fig. 8B, lanes 1-7). Surprisingly, RA was also able to induce the mesodermal Bmp2 and T(Bra) genes even in Srf(-/-) ES cells grown in monolayer, albeit in a delayed fashion. A slight induction of the skeletal Actin gene was also seen in the Srf(-/-) ES cells (Fig. 8B, lanes 8-14). These effects of RA on gene expression profiles of Srf(-/-) ES clones confirmed that these cells had not lost entirely the ability to induce mesodermal genes, but rather exhibited differential gene activation profiles that depended on the nature of the differentiation stimuli.

### EXPERIMENT 9:

### Embryoid bodies developing from Srf(-/-) ES cell aggregates display unusual morphologies.

(A) Scanning electron microscopic depiction of embryoid body morphologies after an in vitro differentiation period of eight days (d8). ES lines used: 99 Srf(-/+) (left), 81 Srf(-/-)(middle), 100 Srf(-/-) (right). The scale bars represent 176 um (left), 300 um (middle),and 231 um (right). Note that fixation does not consistantly preserve EB size.
(B) Immunofluorescent staining of differentiated EBs (d8) for the endodermal surface expression marker laminin using anti-laminin antisera. ES lines used: 99 Srf(-/+) (left), 81 Srf(-/-) (middle), 100 Srf(-/-) (right). The photographic representations depict regional areas of the EBs. Magnification: 400x.

EBs of different Srf genotype and at two stages of differentiation (d5, d8) were sectioned and stained with toluidin to visualize individual cells.
(A) Full views of d8 EBs from ES cells heterozygous or homozygous for the deleted Srf allele. Loose cell packing inside Srf(-/-) EBs is apparent. The ES cells used and their genotypes are indicated. Arrows: visceral endoderm cells; arrowhead: columnar ectodermal cells lining part of a cavity inside the Srf(-/+) EB. Photographs were taken at 200x magnification.
(B) Higher magnification representations of sectioned EBs taken after 5 days (d5; top row) or 8 days (d8; bottom row) of differentiation. Genotypes of ES cells used for EB differentiation were as indicated. The photographic representations depict regional areas of the EBs. The bottom row pictures represent sub-regions of the photographs shown in (A). Photographs were taken at 400x magnification.

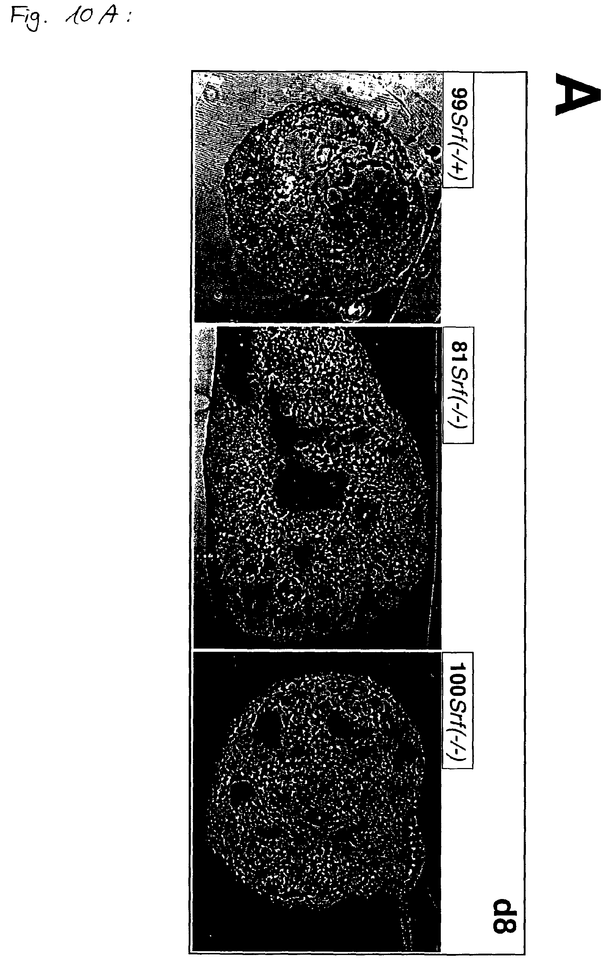

When cultured as cell aggregates, ES cells can differentiate into embryoid bodies (EBs) which contain a wide range of embryonic cell types. "Hanging drop" cultivation of ES cells at high density, under conditions of LIF withdrawal, efficiently initiates EB differentiation. We first investigated the influence of the absence of SRF on the morphology of ESs developing over an 8 day time period. At day 2 of the differentiation period, EBs developed from Srf(-/+) and Srf(-/-) ES cells were of comparable size. From day 2 on, increasing cell death and cell detachment was observed in the Srf(-/-) EBs. Their size was about 40-70% of that of corresponding Srf(+/-) or Srf(+/+) ESs after six days of differentiation, with some variability in size distribution being noticable. Scanning electron microscopy (SEM) of EBs formed from SRF-containing cells revealed smooth homogenous surfaces, indicating tight cell-cell interactions (Fig. 9A, left). This outer cell layer represents visceral endoderm cells (Shen and Leder, 1992, see above). In contrast, Srf(-/-) ES cells formed EBs of irregular structure and heterogenous surface appearance (Fig. 9, middle and right). Such Srf(-/-) EBs displayed two major types of surface structure: first, regions of rough surface, composed of individual cells, each protruding away from neighboring cells and, secondly, smooth patches emerging as large cystic protrusions which by themselves were made up from cells tightly attached to each other. These smooth, cystic protrusions resembled the homogenous appearance of EBs formed by SRF-containing ES cells.

Such cyst-like protruding regions were found with both Srf(-/-) ES cell clones analyzed, although such regions varied with regard to the degree of smoothness. From this analysis it became apparent that the lack of SRF prevented ES cells from forming EBs with homogenous and smooth surfaces indicative of tighly interacting cells. The irregular, heterogenous surface appearance of Srf(-/-) EBs was already discernable at day 5 of EB differentiation. Indirect immunofluorescence staining of day 8 EBs for the presence of the endodermal cell surface marker laminin demonstrated that SRF-containing EBs had differentiated toward the formation of an outer layer of endoderm (Fig. 9B), in all likelyhood visceral mesoderm. In Srf(-/-) EBs this degree of differentiation was not fully realized, except for the segments representing cyst-like protrusions of smooth appearance. This sugggested that the rough surface and lack of tight cell-cell contacts reflected the regional absence of visceral endoderm. A non-uniform differentiation potential within different regions of individual Srf(-/-) EBs is therefore indicated.

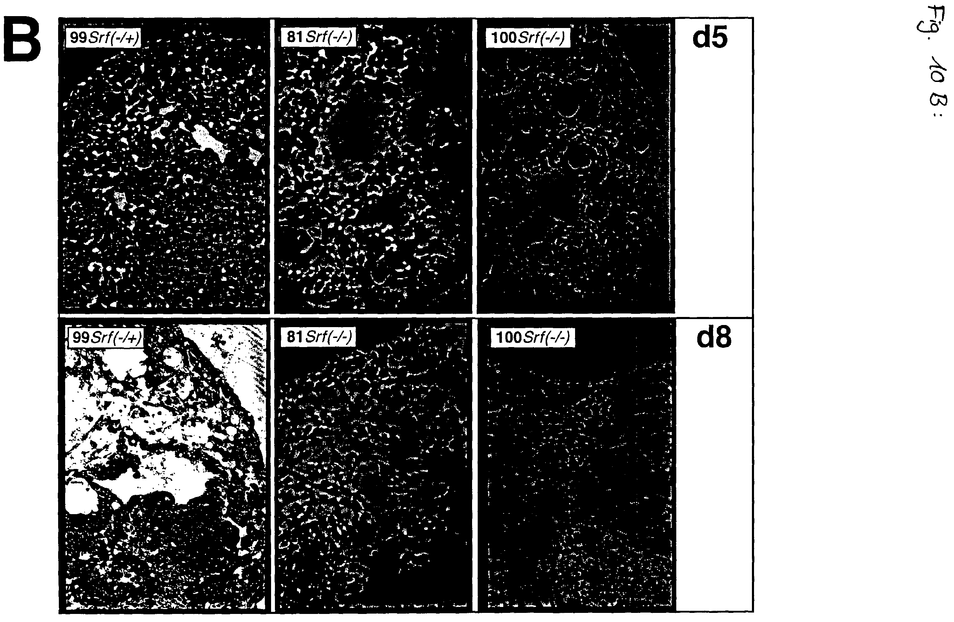

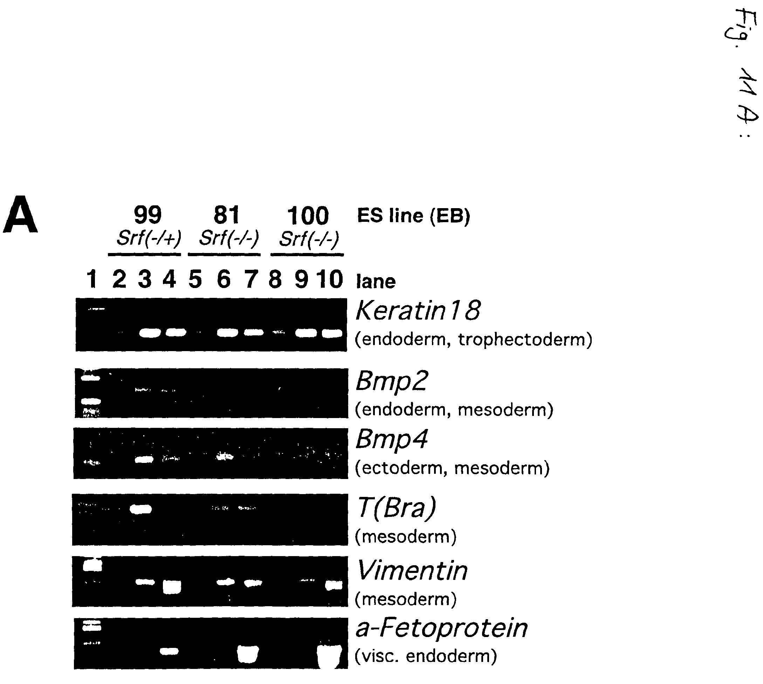

Differentiating EBs develop internal cavities, a process which mirrors proamniotic cavitation of postimplantation mouse embryos (Coucouvanis, E. and Martin, G.R. 1995. Development 126: 535-546). Therefore we next sectioned our differentiating ESs, followed by toluidin staining, in order to inspect changes in internal EB morphology as a function of SRF activity. At day 8 of EB differentiation SRF-containing EBs had not only formed a continuous endodermal layer at the outer rim (see above, and Fig. 10A, left; arrow) but were also in the process of undergoing internal cavitation, with a layer of columnal ectodermal cells lining the emerging cavity (Fig. 10A, left; arrowhead) (Coucouvanis, E and Martin, G.R. 1995, see above). The Srf(-/-) EBs, instead, displayed regional endodermal cells at the FB peripheries in small segments only (Fig. 10A, middle and right) and, in the inside, contained more loosely packed cells and hollow segments. None of these holes appeared lined with columnar epithelial cells. At higher magnification, the difference in cell packing is already clearly visible at d5 of differentiation (Fig. 10B, top row), accompanied by vacuoles present: in SRF-deficient EBs. The higher magnification display of the d EBs (Fig. 10B, bottom row) emphasizes the obvious difference in cell packing.

In summary, at the morphological level, both peripheral and internal cells of EBs displayed altered cell-cell interaction characteristics when formed from ES cells lacking SRF. However, regarding this defect in cell attachment, significant segmental variability was observed within individual Srf(-/-) EBs, suggesting that this impairment can be overcome regionally. Indeed, peripheral regions of smooth appearance also displayed significant expression levels of the visceral endodermal differentiation marker laminin, which was not expressed in the rough EB segments. This suggests that the cellular environment within EBs may provide a regional differentiation stimulus that helps to overcome locally the differentiation impairment of Srf(-/-) ES cells.

### EXPERIMENT 10:

### Embryoid bodies from Srf(-/-) ES cells express mesodermal marker genes.

(A) Semiquantitaiive RT-PCR. EBs, derived from the indicated ES lines (99 Srf(-/+), 81 Srf(-/-), and 100 Srf(-/-) were differentiated for the indicated times (day 2, day 5, or day 8) and subjected to RT-PCR analysis. The marker genes investigated were: Keratin 18 (endoderm, trodhectoderm), Bmp2 (endoderm, mesoderm), Bmp4 (ectoderm, mesoderm), T(Bra) (mesoderm), Vimentin (mesoderm), and α-Fetoprotein (visceral endoderm).
(B) Quantitative RT-PCR. The indicated marker genes (Bmp2, Bmp4, and T(Bra)) were analyzed in EBs identical to those described in (A).

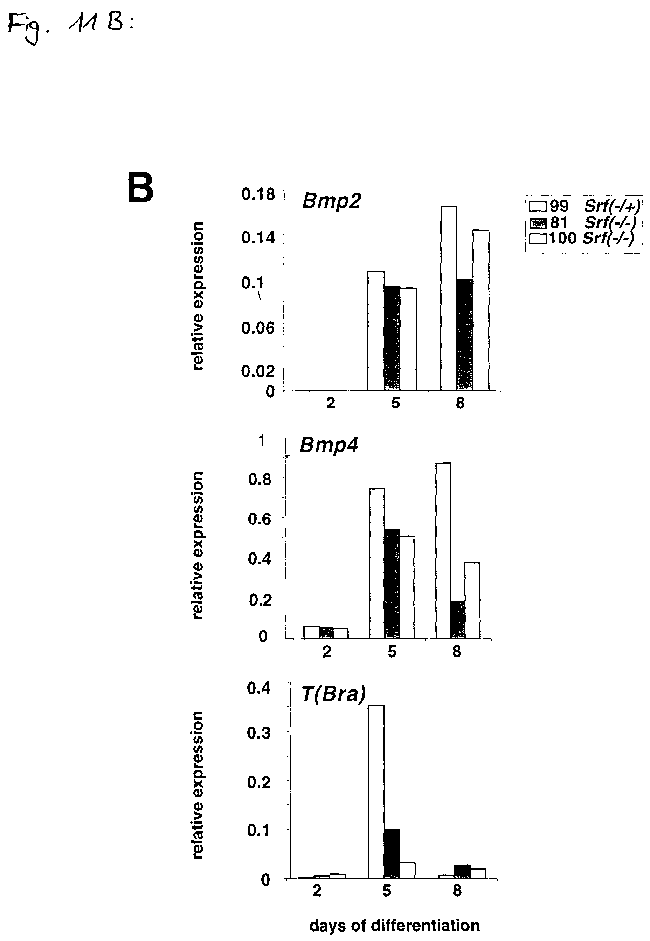

Since both the morphological analysis and the laminin expression data had suggested that Srf(-/-) EBs were able to undergo some degree of differentiation, we reasured the expression levels of other differentiation marker genes in EBs cultivated for up to 8 days in the absence of LIF. Semi-quantitative RT-PCR measurerments revealed not only expression of the endodermnal marker genes Keratin 18 and a-Fetoprotein but also of the mesodermal markers T(Bra), Bmp2, Bmp4, and Vimentin (Fig. 11A). These findings are corroborated by quantitative RT-PCR measurements of the Bmp2, Bmp4 and T(Bra)genes (Fig. 11B). This clearly argues for mesodermal differentiation having taken place to some degree inside Srf(-/-) EBs.

### EXPERIMENT 11:

### Srf(-/-) ES cells retain the ability to develop into mesodermal cell types in vivo.

After subcutaneous injection of Srf(-/-) ES cells into nude mice, the developing teratomas were investigated for the presence of different types of tumor tissue. Several tissues of mesodermal origin, e.g. connective tissue and muscle (A), or cartilage and bone (B) could be identified.

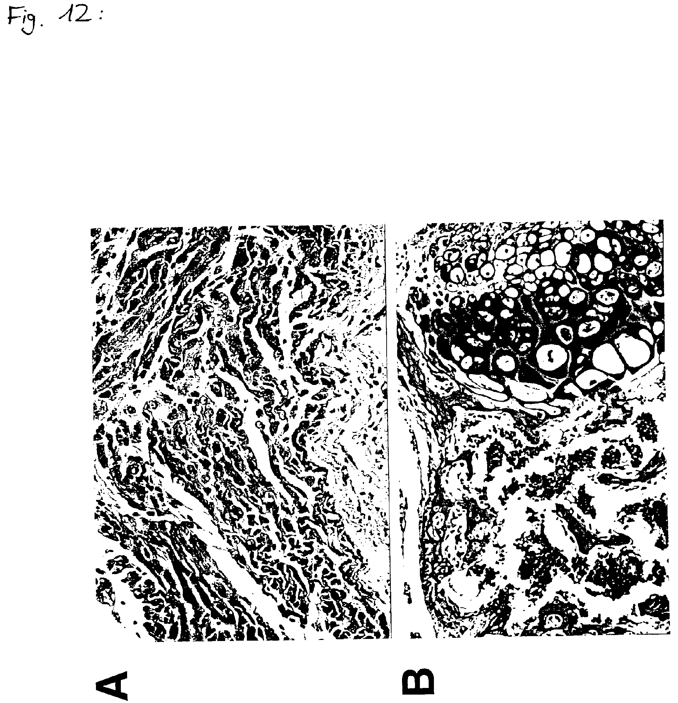

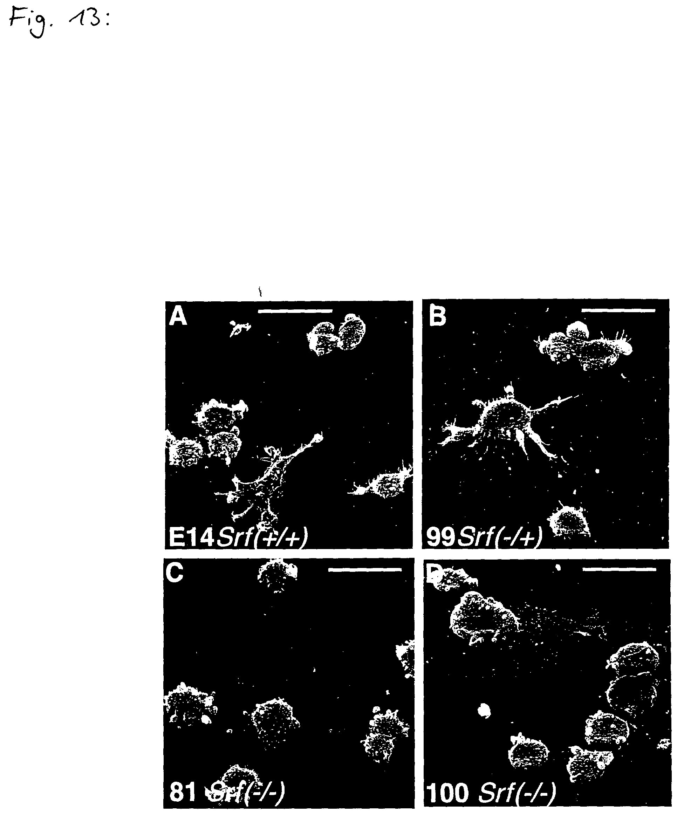

As demonstrated above (Fig. 8B), treatment of monolayer Srf(-/-) ES cells with retinoic acid permitted induction of mesoderm-specific genes like T(Bra), thereby indicating that mesodermal genes could be activated in in vitro cultured Srf(-/-) ES cells by some but not all stimuli. This indicated that the lack of SRF per se does not prevent mesodermal gen activation in general. EBs derived from Srf(-/-) ES cells also displayed patchy expression of mesoderm-specific genes (Fig. 11). We sought to obtain further independent evidence for the mesodermal differentiation potential of ES cells lacking SRF by examining teratomas formed upon injection of Srf(-/-) cells under the skin of nude mice. Here we observed the formation of teratocarcinomas that contained various mesodermal lineage tissues (Fig. 12). We could clearly identify muscle cells and connective tissue (Fig. 12A), as well as bone marrow with hematopoietic cells (including megakaryocytes) and cartilage with chondrocytes (Fig. 12B).

### EXPERIMENT 12:

### Srf(-/-) ES cells display impaired spreading and substrate adhesion.

ES cells of the indicated Srf genotypes were allowed to settle on gelatine-coated tissue culture plates for 2 hours and inspected by scanning electron microscopy. Arrowheads-point to cells engaged in spreading. Scale bar represents 25 µm.
(A) ES cells were grown on gelatine-coated dishes for 48 hours, fixed, and processed for indirect immunofluorescence analysis. Red channel represents staining with antibodies indicated at the right of the figure. Green channel represents staining with Oregon-green conjugated phalloidin. Genotypes of the cell lines used are indicated. (B) Analysis of expression of focal adhesion components by Western blotting. ES cell extracts were resolved by SDS-PAGE, blotted and the membranes probed with antibodies against FAK β-actinin, and vinculin.

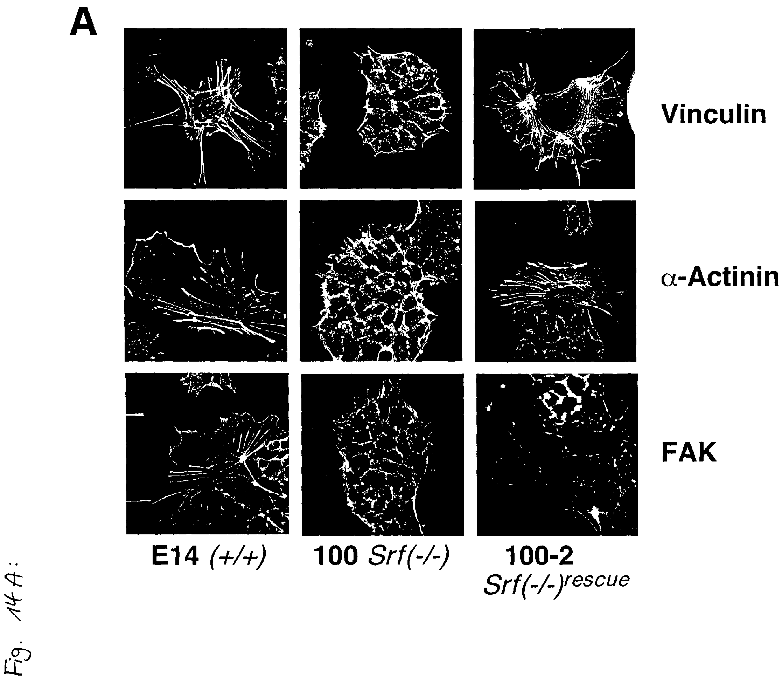

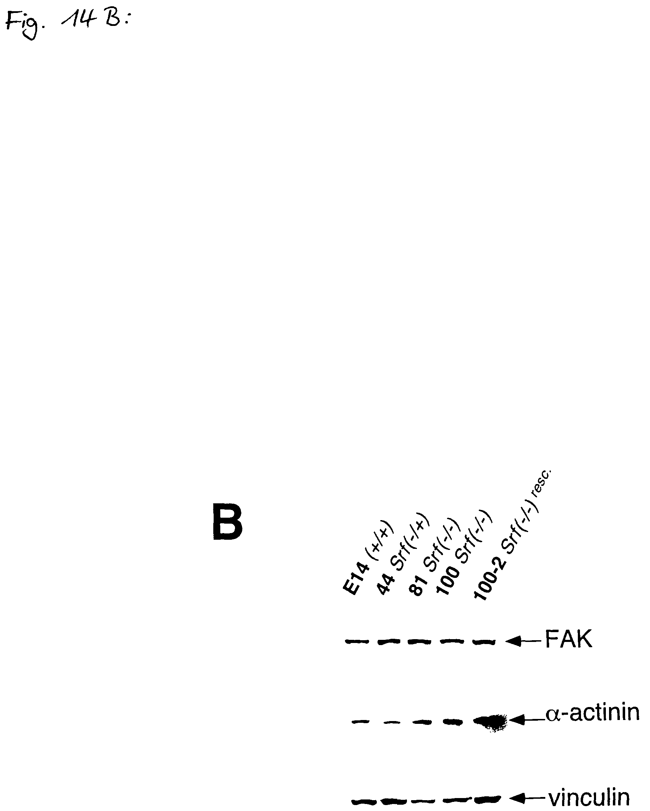

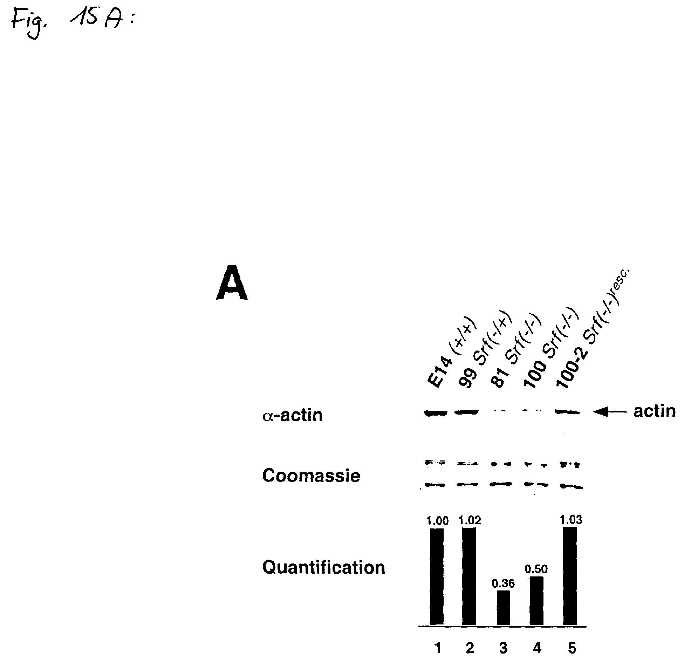

Plating of undifferentiated wildtype or heterozygous Srf(-/+)ES cells on gelatine-coated tissue culture plates, in the presence of LIF, resulted in spreading of about 20% of the cells after 2 hours (Fig. 13A, B), in agreement with previous reports (Priddle, H., Hemmings, L., Monkley, S., Woods, A., Patel., B., Sutton, D., Dunn, G.A., Zicha, D. and Critchley, D.R. 1998. J Cell Biol 142: 1121-1133). In contrast, cells of two independent Srf(-/-) ES cell lines remained rounded under these conditions, without any signs of cell surface projections (Fig. 13C, D). On fibronectin, Srf(-/-) ES cells were able to spread, although more slowly and to a lesser extent than wildtype ES cells. Adhesion to laminin was more affected by the lack of SRF than adhesion to fibronectin or collagen IV. The formation of specialized F-actin structures, like stress fibers and lamellipodia, is required for a cell to spread. We therefore analyzed wildtype, Srf(-/-) and Srf(-/-) rescue ES cells for their abilities to form focal adhesions and associated actin stress fibers. 48 h after plating on either fibronectin or gelatine, wildtype and Srf(-/-)^{rescue} ES cells assembled numerous actin stress fibers that terminated in focal adhesions which stained positive for vinculin, FAK and actinin (Fig. 14A, left and right panels). In contrast, in Srf(-/-) ES cells F-actin staining was limited to cell margins and was rather diffuse (Fig. 14A, middle panel). In addition, staining for the focal adhesion proteins vinculin, FAK and β-actinin was limited to the cell margins and did not show the typical punctate pattern. In the case of vinculin, however, some punctate staining could be observed at the periphery of Srf(-/-) ES cell colonies. The use of fibronectin as growth substrate did not enable assembly of focal adhesions or stress fibers in Srf(-/-) ES cells, as was also seen previously with Talin (-/-) ES cells (Priddle et al.,1998, see above). The failure of Srf(-/-) ES cells to assemble focal adhesions was not due to a general defect regarding the expression of the focal adhesion components FAK, actinin, and vinculin, as judged by Western blotting(Fig. 14B). We note however, that protein levels expressed from the SRF target gene Vinculin were reduced in Srf(-/-)ES cells by 20-40% (Fig. 14B). We conclude from these data that undifferentiated Srf(-/-) ES cells are severely impaired in undergoing cell-substrate interactions, cell shape dynamics, and spreading activity. These defects, due to SRF deficiency, correlate with severe impairments in building up stress fiber arrays and associated focal adhesion plaques.

### EXPERIMENT 13:

### Srf(-/-) ES cells display reduced overall actin expression and an altered ratio of F-versus G-actin

(A) Western blot analysis of total cellular actin protein in ES cells. Whole cell extracts from ES cells of the indicated Srf genotypes were separated by SDS-PAGE, blotted and probed with a pan-actin antiserum (upper panel). Coomassie staining of the gel after blotting confirms equal loading (middle panel). Densitometric quantification of the actin signals is shown in the lower panel, whereby the signal from the E1 4.1 Srf(+/+) wildtype cells was arbitrarily set to one.
B) Histological distributions of F-actin and E-cadherin in cell colonies derived from ES cells of different Srf genotype. Wildtype cells (left panel) and the two Srf(-/-) cell lines 226-81 (middle panel) and 226-100 (right panel) were grown on coverslips for 48 hours. Expression of F-actin (upper row) and E-cadherin (lower row) was detected with Texas-Red phalloidin and an E-cadherin-specific antiserum, respectively.
C) Quantitation of actin isoforms by flow cytometry. 99 Srf(-/+) ES cells (red), and Srf(-/-) ES lines 81 (orange) and 100 (blue), were stained with Oregon-Green Phalloidin (upper panel) and Texas-Red DNAsel conjugate (bottom panel) to specifically stain for F-Actin and G-Actin, respectively. Fluorescence intensity values are given on the abscissae.

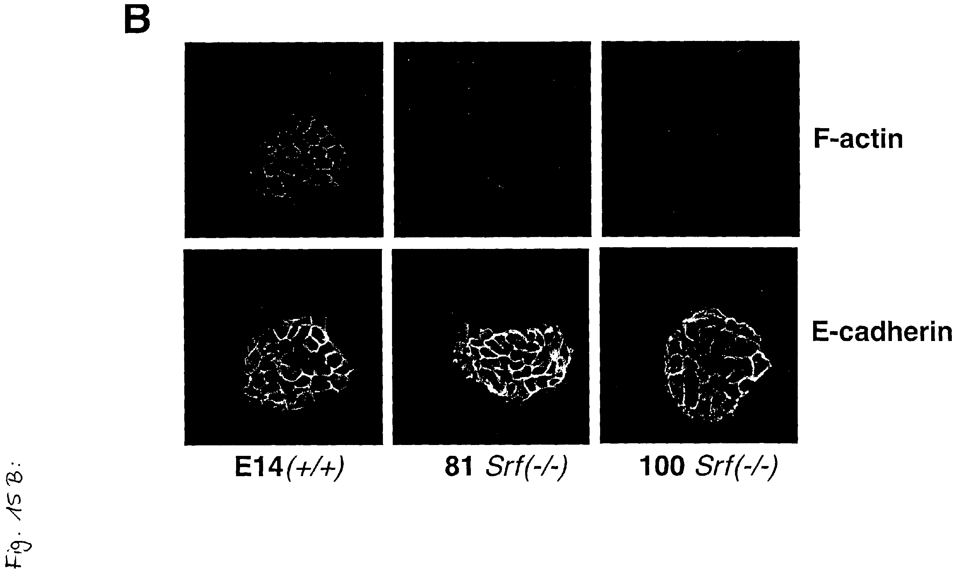

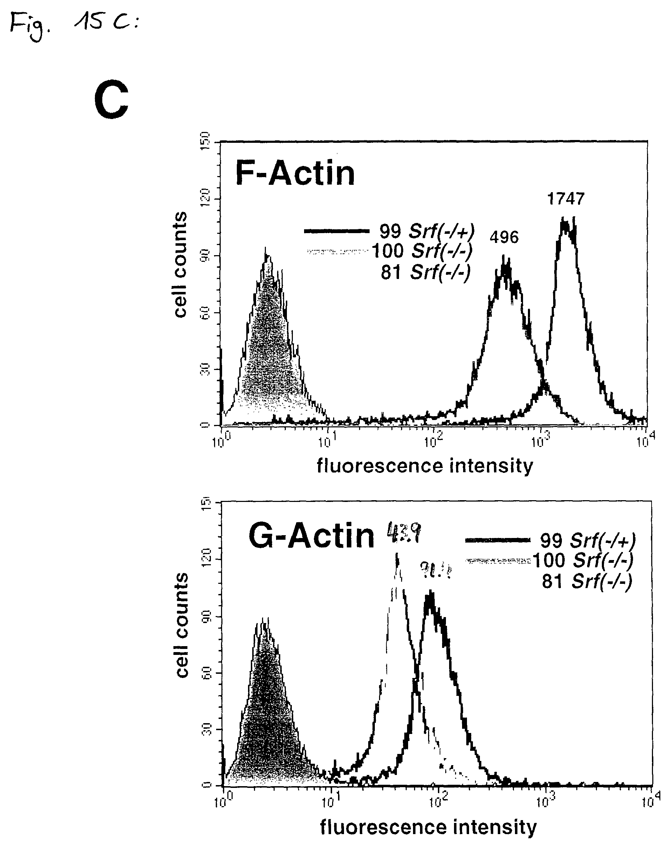

Regulation of Actin gene expression by SRF has been studied extensively. Functional SREs have been described for the genes encoding α-, β and γ-Actins (Browning, C.L., Culberson, D.E., Aragon, I.V., Fillmore, R.A., Croissant, J.D., Schwartz, R.J. and Zimmer, W.E. 1998. Dev Biol 194: 18-37; Chen, C.Y., Croissant, J., Majesky, M., Topousis, S., McQuinn, T., Frankovsky, M.J. and Schwartz, R.J. 1996. Dev Genet 19: 119-130; Frederickson, R.M., Micheau, M.R., Iwamoto, A. and Miyamoto, N.G. 1989. Nucleic Acids Res 17: 253-270). In Srf(-/-) ES cells, Western blotting revealed a 2-3 fold decrease in overall actin protein levels in Srf(-/-) ES cells compared to those of either wildtype, heterozygous, or Srf(-/-)^{rescue} ES cells (Fig. 15A). Staining of F-actin using phalloidin showed a typical cortical actin network in non-spread wildtype ES cell colonies (Fig. 15B, left panel). In colonies formed by cells of two independent Srf(-/-) ES cell lines, the cortical actin was severely reduced (Fig. 15B, middle and right panel). Expression and localization of E-cadherin, on the other hand, appeared unaffected by SRF (Fig. 15B, lower row). We next used FACS analysis for quantitative measurements of the actin polymerization state inside cells. In this assay, a 3.5-fold reduction of F-actin levels was observed in Srf(-/-)ES cells (Fig. 15C, upper panel). G actin levels, as determined by DNAse I staining, were affected maximally twofold (Fig. 15C, lower panel). In summary, these results document a role for SRF both in the regulation of overall actin expression levels and, probably indirectly, in actin treadmilling.

### EXPERIMENT 14:

### Zyxin gene expression is dependent upon SRF

(A) Atlas array mRNA expression analysis of ES cell lines 44 Srf(-/+) and 100 Srf(-/-). A section of the entire mouse Atlas array, consisting of 588 different genes spotted in duplicate, is, shown. Arrowhead denotes the duplicate signal for zyxin cDNA, which does not appear in 100 Srf(-/-) and 81 Srf(-/-) hybridizations.
B) The panel of housekeeping genes present on the Atlas array is shown to confirm comparable cDNA input. Note the disparate expression of the β-Actin gene (signal 4) in the two cell lines tested. Further housekeeping genes: (1) Ubiquitin, (2) Glyceraldehyde-3-phosphate dehydrogenase, (3) Ornithine decarboxylase, and (5) Ribosomal protein S29. Similar results were obtained when the hybridization was performed with cDNA of ES 99 Srf(-/+) versus ES 81 Srf(-/-).
(C) Total Zyxin mRNA levels in ES cells. Quantitative RT-PCR using Zyxin specific primers. Relative mRNA measurements for the indicated ES cell lines were performed as described in Material and methods using zyxin specific primers.
(D) Western blot analysis of zyxin expression in ES cells. Whole cell extracts of the indicated cell lines were resolved by SDS-PAGE, blotted, and probed with zyxin specific antiserum. No zyxin protein is detectable in the two Srf(-/-) ES cell lines tested.
(E) Average induction levels of Zyxin mRNA after 3 hours of serum treatment, as monitored by quantitative RT-PCR. Indicated ES cell lines were serum-starved and treated with 15 % FCS for 180 minutes. Values were normalized to Hprt mRNA levels and the uninduced value was set to one. Standard deviations refer to the mean of five independent experiments.
(F) Indirect immunofluorescence analysis of zyxin expression in ES cell colonies. ES cells of the indicated Srf genotype were grown for 48 hours on gelatine-coated coverslips, fixed and stained with zyxin-specific antiserum (red) together with Oregon green-phalloidin (green) to visualize filamentous actin. In the case of wildtype or Srf(-/-)^{rescue} ES cells, zyxin expression is detected in focal adhesion of spread individual cells (upper left or lower right) or at the margins of cells within a colony (upper right). Almost no zyxin expression is found in Srf(-/-) ES cell colonies (lower left). Ectopic SRF expression in Srf(-/-)^{rescue} cells reestablishes Zyxin expression at focal adhesions (lower left).

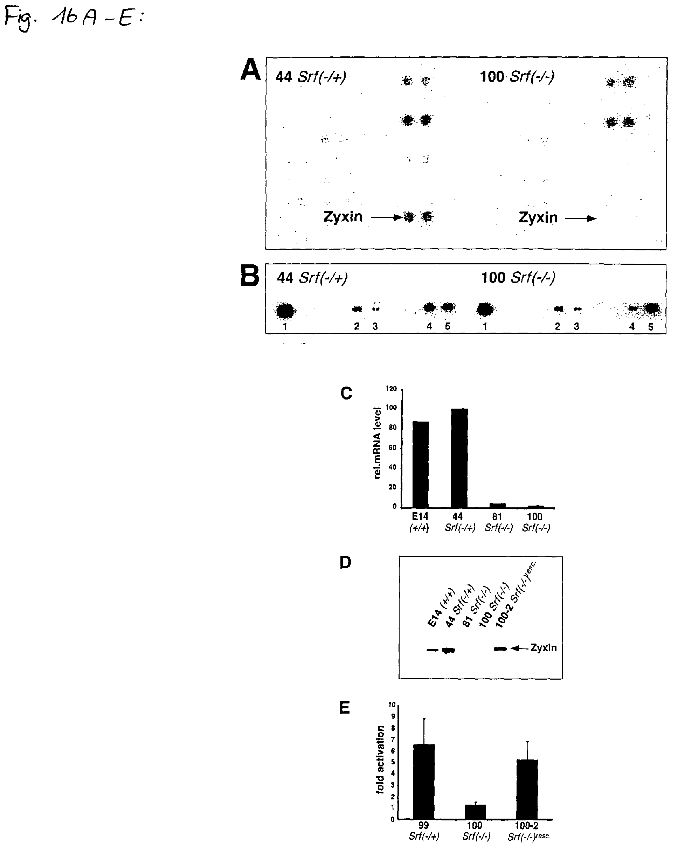

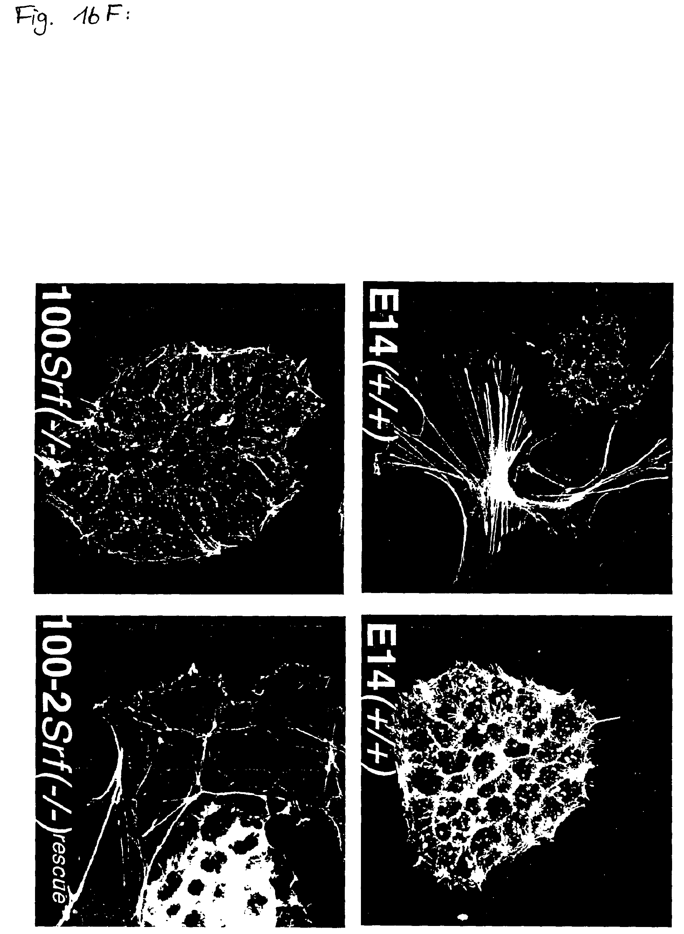

SRF, being a transcription factor, most likely exerts its effects through the regulation of gene expression. For a broader look at changes in mRNA expression profiles as a function of SRF deficiency, we used Atlas array membranes, representing 588 different genes. Two membranes were incubated in parallel with radioactively labeled cDNA of the ES cell lines 44 Srf(-/+) and 226 100 Srf(-/-), respectively, and signals were visualized by phosphoimaging (Fig. 16A, B). Comparable and reproducible resuls were obtained in a different hybridization experiment using the ES cell lines 226-99Srf(-/+) and1226-81 Srf(-/-). Among other genes, Zyxin mRNA was down regulated in both independently derived SRF-deficient ES cell lines (Fig. 16A). Equal hybridization efficiency of the used membrane pairs was demonstrated using a variety of housekeeping genes (Fig. 16B), whereby downregulated Actin expression provided an internal control for a known SRF target gene. SRR-dependent differential expression of Zyxin at mRNA and protein levels in undifferentiated ES cells was subsequently confirmed by quantitative RT-PCR and Western blotting, respectively (Fig. 16C, D). The IEGs c-fos and Egr-1, as well as the genes encoding the cytoskeletal proteins vinculin and -actin, have previously been shown to be serum-inducible. We have demonstrated that such IEG responses are dramatically reduced in the absence of SRF (see above). These observations raised the possibility that Zyxin also belonged to the set of genes rapidly induced upon serum stimulation. ES cells of different Srf genotype were therefore cultured for 36 hours in the absence of serum, followed by addition of 15% serum and RNA extraction after 0, 10, 30, 60 and 180 min. Zyxin mRNA levels increased gradually with time after treatment of Srf(-/+) and Srf(-/-)^{rescue} ES cells, reaching the maximum after 180 min (Fig. 16E). In contrast, almost no Zyxin induction was observed for the Srf(-/-) ES cell clones tested (Fig. 16E). Serum induction for 180 min resulted in a 5-6 fold increase in Zyxin mRNA for Srf(-/+) and Srf(-/-)^{rescue} ES cells and an only 1-2 fold increase for Srf(-/-) ES cells (Fig. 16E). These measurements identify Zyxin as a novel serum-inducible gene, the induction of which is strictly dependent upon the presence of SRF. The kinetics of induction are comparable to those of other cytoskeletal genes, like Actin and Vinculin. In somatic cell lines, zyxin has been primarily found in focal adhesions and other sites of high actin assembly (Beckerle, M.C. 1997. Bioessays 19: 949-957). To monitor zyxin localization in ES cells, we cultured ES cells on gelatine-coated coverslips for 48h. Cells were fixed and indirect immunofluorescence was performed with anti-zyxin antisera. FITC-phalloidin was included to visualize actin filaments. In agreement with previous studies using somatic cells, zyxin was concentrated at focal adhesion plaques in wildtype ES cell colonies that had undergone spreading after 48h (Fig. 16F, upper left). Prominent actin stress fibers that emanated from these sites were also clearly visible. In wildtype colonies that had not yet spread, zyxin colocalized with cortical actin at the cell margins (Fig. 16F, upper right). In contrast, expression of zyxin was drastically reduced in homozygous mutant ES cell colonies (Fig. 16F, lower left). Also, the residual zyxin expression pattern was clearly distinct from those observed for FAK, -actinin and vinculin in Srf(-/-) ES cell colonies (compare Fig. 16F to Fig. 14A). Reintroduction of SRF expression vectors into Srf(-/-) cells (i.e. Srf(-/-)^{rescue} cells) regained Zyxin expression at focal adhesions (Fig. 16F, lower right). Altogether, the defect of Srf(-/-) ES cells in the formation of adhesion plaques and associated actin stress fibers coincides with downregulation of the focal adhesion protein zyxin. However, zyxin alone is not sufficient to promotefocal adhesion formation in ES cells, since transient overexpression of zyxin in Srf(-/-) ES cells did not rescue the defective actin cytoskeleton.

### EXPERIMENT 15:

### Constitutively active SRF (SRF-VP16) induces molecular and cell morphological changes in ES cells of Srf(-/-) genetic background.

(A) Schematic representation of the various SRF coding sequences expressed upon transient transfection of ES cells. In SRF-VP16, part of the SRF transactivation domain (TAD) is replaced by the HSV VP16 activation domain. In the control construct SRF M-VP16, the major part of the MADS-box is removed. NLS nuclear localization signal.
(B) Induction of Egr-1, Vinculin, Zyxin and β-Actin at the transcriptional level. RT-PCR analysis was performed on ES cell extracts of the indicated genotypes three days after transfection with either empty vector, SRFΔM-VP16 encoding vector. Relative mRNA levels for each sample were calculated as described in Materials and methods. Values are given as fold induction over cells transfected with empty vector only. Comparable expression levels of the SRF effector proteins was confirmed by quantitative Western blotting. A representative data set of two independent experiments is shown.
(C) SRF-VP 16 expression leads to increased stress fiber formation. 100Srf(-/-) ES cells were transiently transfected with SRF-VP16 (upper panel) and the SRFΔM-VP16 control construct (lower panel) and processed for immunofluorescence analysis three days later. Cells were simultaneously stained with phalloidin (green) and anti-VP16 (red).

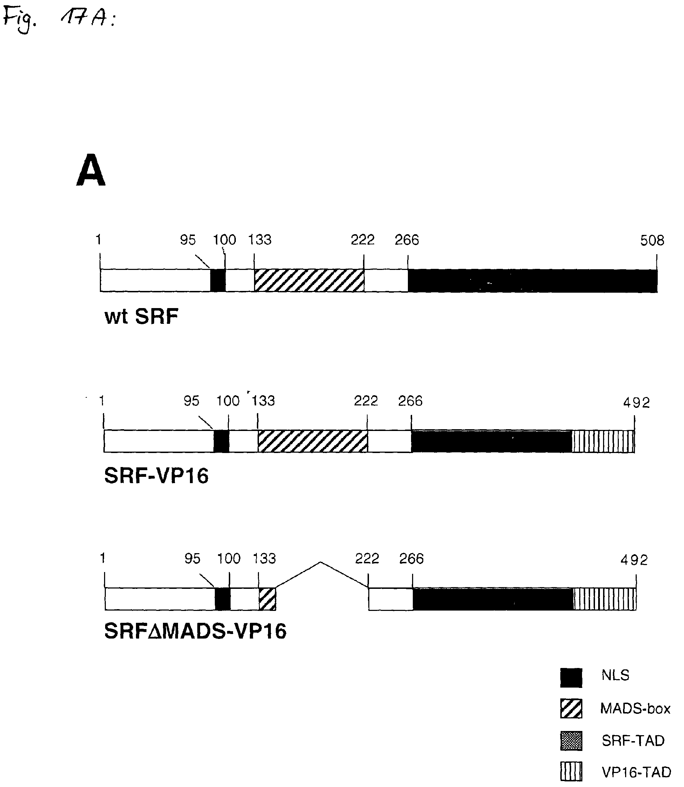

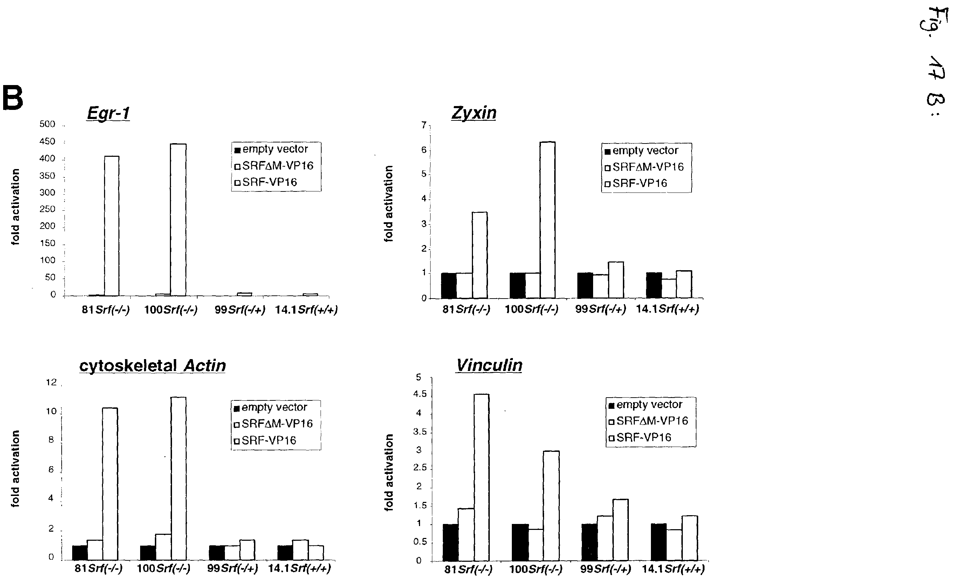

SRF-VP16 fusion proteins were previously used to drive SRF-dependent transcription independent of upstream signalling cascades (Hines, W.A., Thorburn, J. and Thorburn, A. 1999. Mol Cell Biol 19: 1841-1852; Johansen, F.E. and Prywes, R. 1994. Mol Cell Biol 14: 5920-5928; Treisman, R. Marais, R. and Wynne, J. 1992. EMBO J 11: 4631-4640). Such constitutively active SRF should reveal an existing link between SRF activity and the status of the cytoskeleton. To control for potential unspecific effects of the VP16 activation domain, we constructed an SRFΔM-VP16, which lacks part of the MADS box, consequently being unabbe to dimerize and bind to SREs, yet retaining the ability to locate to nuclei (Fig. 17A). Undifferentiated ES cells were transiently transfected with SRF-VP16 and SRFΔM-VP16 expression vectors, and RNA expression levels were measured by quantitative RT-PCR (Fig. 17B).In an Srf(-/-) background, the mRNA levels for the IEGs Egr-1 (Fig.17B, upper left) and c-fos were increased about 400-fold by introduction of SRF-VP16 ascompared to mock-transfected cells, confirming the capacity of SRF to drive expression of those endogenous genes. Importantly, introduction of SRFΔM-VP16 had no significant effect. Zyxin mRNA levels were specifically induced 3-6-fold by SRF-VP16 (Fig. 17B, upper right), similar to the known SRF-regulated genes encoding the cytoskeletal proteins vinculin and β-actin (Fig. 17B, lower panel). SRF is therefore identified as an essential regulatory protein which drives the expression of several cytoskeletal proteins, including zyxin. Interestingly, introduction of SRF-VP16 into Srf(-/-) ES cells led to dramatic cell morphological changes characterized by cell flattening, whereas cells transfected with the control construct looked unaltered. We speculated that SRF-induced cytoskeletal rearrangements might be responsible for these changes in cell morphology. Indeed, staining for F-actin revealed the formation of large arrays of stress fibers (Fig. 17C, upper) and frequent occurrence of lamellipodia (not shown) in each SRF-VP16 transfected Srf(-/-) ES cell. The cytoskeleton of SRF DM-VP16 transfected Srf(-/-) ES cells did not undergo any detectablechanges (Fig. 17C, lower). Simultaneous staining with anti-VP16 antibody, as well as Western blotting (not shown), confirmed that SRF-VP16 and SRFΔM-VP16 were expressed at comparable levels and that both proteins accumulated in the cell nuclei. Interestingly, the effects elicited by expression of constitutively active SRF on the expression of target genes (Fig. 17B) and ES cell morphology were much less pronounced in ES cells of Srf(+/+) or Srf(-/+)genetic background. Endogenous SRF protein present in these cell lines probably competes with SRF-VP16 for binding tocognate promoters. Reintroduction of wildtype SRF protein also had only minor effects on gene expression and cytoskeletal rearrangements. These data demonstrated conclusively that constitutively active SRF is able to elicit drastic cell morphological and molecular changes that are linked to remodelling of the actin cytoskeleton.

### EXPERIMENT 16:

### Reduced cell-cell interaction in multicellular embryoidbodies (EB) derived from Srf(-/-) ES cells.

(A) Scanning electron microscopy of day 8 EBs generated of either heterozygous (upper panel) or homozygous (lower panel) Srf mutant ES cells. Scale bar represents 20 µm.
(B) Expression of cytoskeletal proteins in cells at EB surface areas. Indirect immunofluorescence was performed with day 8 EBs of either wildtype or Srf(-/-) genotype. Proteins to which antibody reagents were directed are inidcated at the right. Top row: vinculin (red), F-actin (green); bottom row: zyxin (red), β-catenin (green). Co-localization of proteins is reflected by the yellow color.

Ultrathin sections were performed on day 8 EBs derived from ES cells of the indicated Srf genotypes, and inspected by transmission electron microscopy. Zones of cellcontact, where actin filaments are enriched in Srf(-/+) EBs but less so in Srf(-/-) EBs, aremarked by arrowheads. Segments of impaired cell-cell adhesion are apparent in the Srf(-/-) EBs between the arrows. Scale bar represents 1 µm.

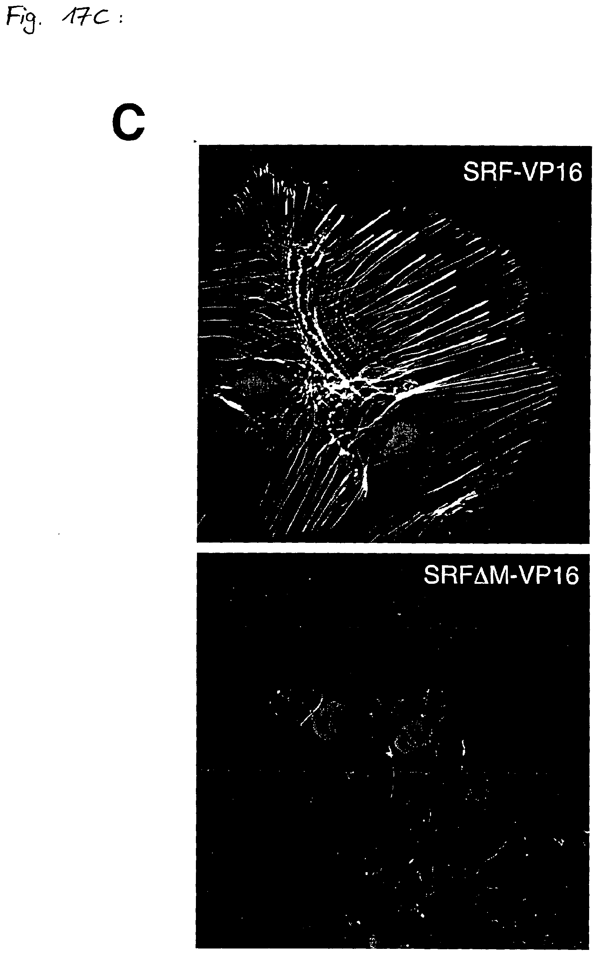

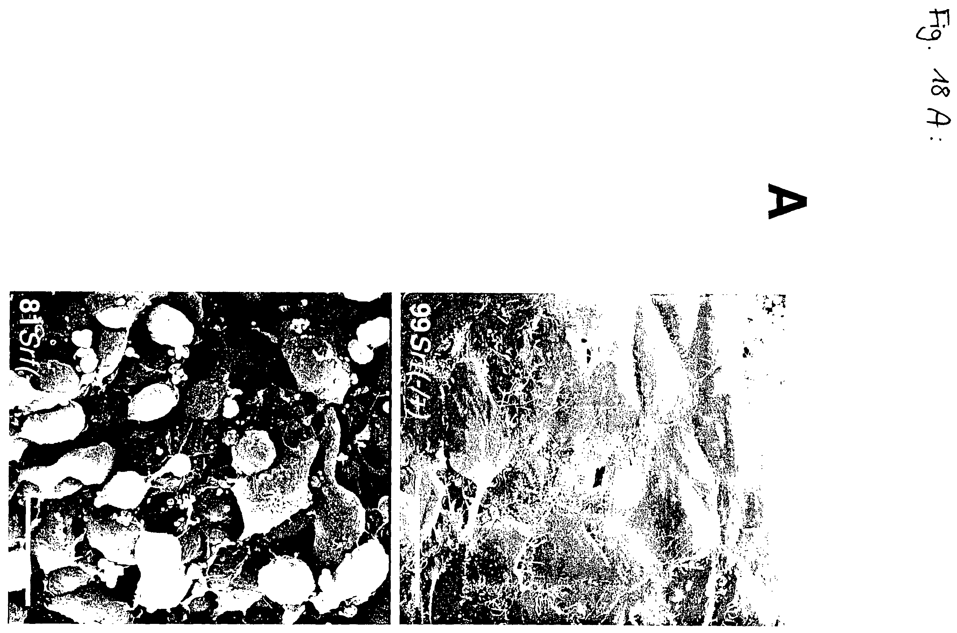

To extend our analysis concerning the role of SRF-dependent cytoskeletal activities from individual cells to a multicellular system, we resorted to embryoid body (EB) differentiation. EBs form upon differentiation of ES cell aggregates ex vivo (Drab, M., Haller, H., Bychkov, R., Erdmann, B., Lindschau, C., Haase, H., Morano, I., Luft, F.C. and Wobus, A.M. 1997. Faseb J 11: 905-915). Like in the embryo, the visceral endoderm (VE) represents the first epithelium that forms on the outer surface of an EB. In order to investigate EB cellular morphology in detail, EBs differentiated for eight days (d8) were fixed and inspected by scanning electronmicroscopy. At d8, the VE surface of heterozygous EBs was very smooth. The outer cells formed a closely packed and well differentiated epithelial structure (Fig. 18A, upper panel). In EBs derived from Srf(-/-) ES cells, however, cells at the surface were not flattened and only loosely packed, indicating impaired adhesion between cells. This resulted in a very rough surface morphology, reminiscent of poorly differentiated endoderm (Fig. 18A, lower panel). A recent report demonstrated the importance of the actin cytoskeleton for the formation of adherens junctions in epithelial (Vasioukhin, V., Bauer, C., Yin, M. and Fuchs, E. 2000. Cell 100: 209-219). Therefore it seemed possible that defective cell-cell interaction in the Srf(-/-) EBs was a result of altered localization of adherens junction components. Vinculin and Zyxin have been previously described to localize to adherens junctions. Immunofluorescence of wildtype d8 EBs revealed a perfect colocalization of F-actin and vinculin at the cell margins of epithelial cells. Additionally, numerous focal contacts were labeled with the vinculin antibody (Fig. 18B, upper left). In EBs derived from Srf(-/-) ES cells, however vinculin expression was drastically reduced and the protein was distributed throughout the cytoplasm (Fig. 18B, upper right). No colocalization of vinculin and F-actin occured at the cell margins, suggesting that Srf(-/-) ES cells are unable to form vinculin-containing adhesion complexes. The localization of zyxin in EBs developed from wild type ES cells was similar to that of vinculin (Fig. 18B, lower left).In Srf(-/-) EBs, however, anti-zyxin labeled only few focal contacts and staining at the cell periphery was totally absent (Fig.18B, lower right). β-catenin, a well established component of adherence junctions, was unaltered in its localization (Fig. 18B, right panel). Therefore, vinculin and zyxin are strongly affected in both their expression and localization in EBs generated from Srf(-/-) ES cells.

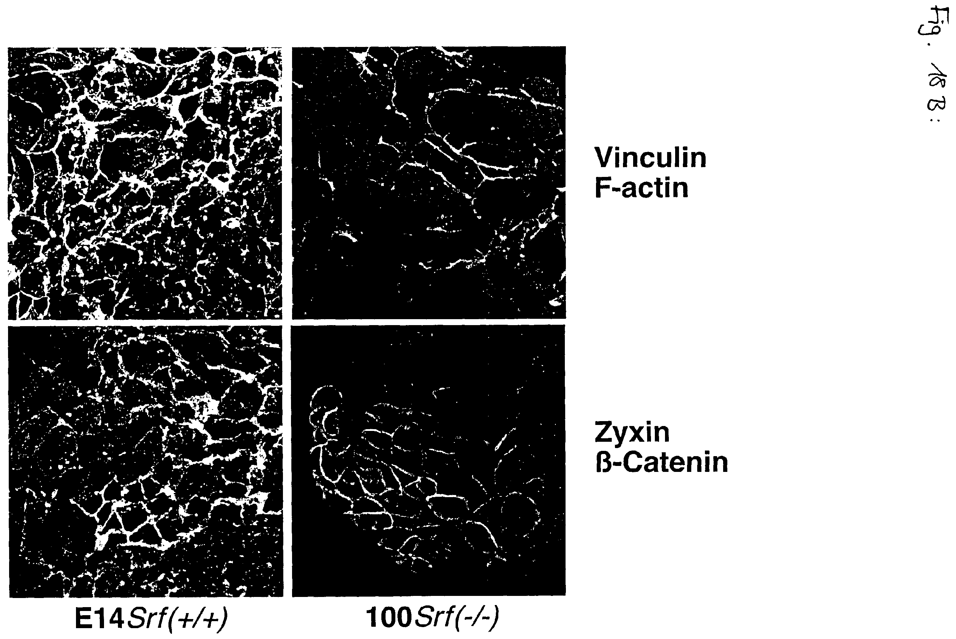

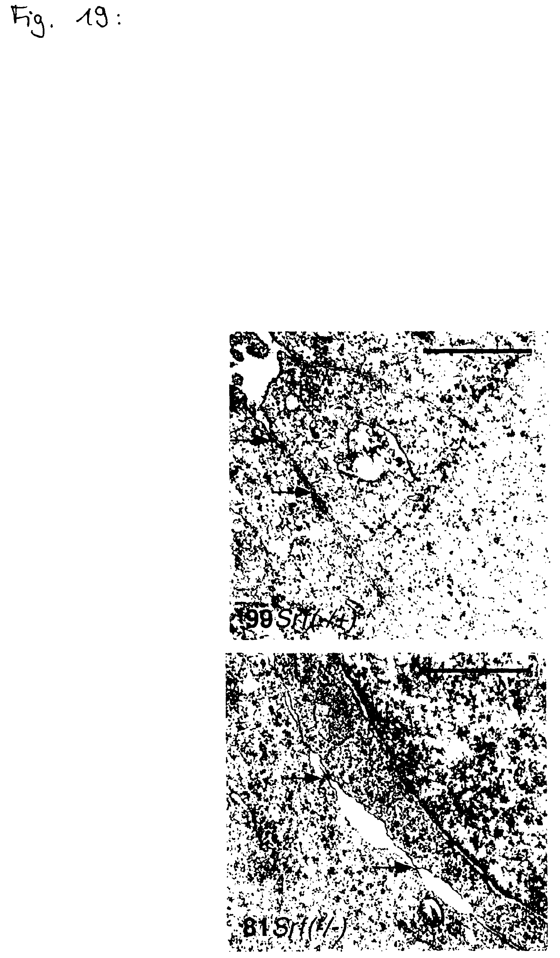

Sectioning of EBs and subsequent electron microscopic inspection allowed a more detailed visualization of regions of cell-cell interaction. Srf(-/+) ES cells inside EBs display intimate contact, and actin filaments are enriched in the zones of intercellular adhesion (arrowhead in Fig. 19, upper panel). In contrast, large gaps and spaces are prominent between differentiated ES cells lacking SRF (Fig. 19, lower panel). Actin filaments were also clearly diminished at sidesof cell cell contact in comparison to wildtype or heterozygous cells (arrowheads in Fig. 19).

Thus, intercellular structures built up of filamentous actin, probably representing adherence junctions, are established inefficiently between differentiated cells of Srf(-/-) genotype, resulting in fewer cell-cell contacts. The SRF-controlled genes zyxin and vinculin are likely involved in the process, since delocalization of their protein products correlates with defective cell-cell adhesion.

## Claims

1. Use of an active agent influencing, particularly stimulating the expression and/or function of SRF, SRF variants and/or members of the SRF signal transduction pathway in eukaryotic cells, for the preparation of a therapeutic drug or a pharmaceutical composition for the treatment of disturbances or illnesses that are linked with SRF-related cellular malfunctions.

2. Use of a substance detecting the expression and/or function of SRF, SRF variants and/or members of the SRF signal transduction pathway in eukaryotic cells, for the diagnosis of disturbances or illnesses that are linked with SRF-related cellular malfunctions.

3. Use according to claim 1 or 2, **characterized in that** said SRF-related cellular malfunction is a malfunction of structural components of the cellular cytoskeleton.

4. Use according claim 3, **characterized in that** said structural components of the cytoskeleton are focal adhesion plaques, stress fibers, adherence junctions and/or cytoskeletal actin network.

5. Use according to one of the preceding claims, **characterized in that** said members of the SRF signal transduction pathway are the genes Arl-4, Bcl-2, Bmp2, Bra(T), Cathepsin B, Cathepsin L, IGFBP-6, Integrin , Keratin 16, Keratin 18, LIM-K2, MKK6, gene for Oxidative stress-induced protein, TDAG15, TGF -R type 1, uPA-R and Zyxin.

6. Use according to one of the preceding claims, **characterized in that** said disturbances or illnesses at least partly result from altered cell migration processes.

7. Use according to one of the preceding claims, **characterized in that** said disturbances or illnesses are tumor invasion, tumor metastasis, auto-immune diseases, disturbances of wound healing, lymphocyte homing and disturbances of immune defense mechanisms.

8. Use according to one of the preceding claims, **characterized in that** said active agent or substance is targeted against SRF, SRF variants and/or one member of the SRF signal transduction pathway.

9. Use according to one of the preceding claims, **characterized in that** said active agent or substance is targeted against activators, inhibitors, regulators and/or biological precursors of SRF, SRF variants and/or members of the SRF signal transduction pathway.

10. Use according to one of the preceding claims, **characterized** n that said active agent or substance is a polynucleotide encoding a peptide, preferably a polypeptide, which influences, preferably stimulates the expression of SRF, SRF variants and/or members of the SRF signal transduction pathway.

11. Use according to one of the preceding claims, **characterized in that** said active agent or substance is a peptide, preferably a polypeptide, which influences, preferably stimulates the expression of SRF or SRF variants and/or members of the SRF signal transduction pathway.

12. Pharmaceutical composition, comprising an effective amount of at least one active agent influencing, preferably stimulating the expression and/or function of SRF, SRF variants and/or members of the SRF signal transduction pathway in eukaryotic cells, and optionally further comprising a pharmaceutically acceptable carrier.

13. Pharmaceutical composition, comprising an effective amount of at least one active agent influencing, preferably stimulating the expression and/or function of activators, inhibitors, regulators and/or biological precursors of SRF, SRF variants and/or members of the SRF signal transduction pathway in eukaryotic cells, and optionally further comprising a pharmaceutically acceptable carrier.

14. Pharmaceutical composition according to claim 12 or 13, comprising an active agent as defined in one of the claims 10 or 11.

15. Diagnostic kit, comprising at least one substance detecting the expression and/or function of SRF, SRF variants and/or members of the SRF signal transduction pathway in eukaryotic cells.

16. Genetically engineered embryonic or somatic stem cell, modified at the Srf locus, preferably by complete or in part deletion of one, especially both Srf alleles or by replacement of one, especially both Srf alleles or with mutated versions of SRF encoding SRF proteins with altered activity, or a cell derived from said cell.

17. Method for the screening of new SRF target genes, **characterized in that** stem cells according claim 16 are used.

18. Method for screening and identifying an active agent or substance to be used according to one of claims 1 to 15, **characterized in that** the influence of compounds on the expression and/or function of SRF, SRF variants and/or members of the SRF signal transduction pathway is tested.

19. Method according to claim 18, **characterized in that** stem cells according claim 16 are used.
